# EUROPEAN PATENT SPECIFICATION

(11) **EP 3 302 219 B1**
(45) Date of publication and mention of the grant of the patent: **03.07.2024**
(21) Application number: 16799477.1
(22) Date of filing: 25.05.2016
(51) Int. Cl.: A61B 1/00

(54) **DISTAL FRONT END FOR COORDINATED POSITIONING OF AN ENDOSCOPE WITH A SUCTION DEVICE**
DISTALES FRONTEND ZUR KOORDINIERTE POSITIONIERUNG EINES ENDOSKOPS MIT EINER SAUGVORRICHTUNG
EXTRÉMITÉ AVANT DISTALE POUR LE POSITIONNEMENT COORDONNÉ D'UN ENDOSCOPE AVEC UN DISPOSITIF D'ASPIRATION

(30) Priority: 27.05.2015 US 201514722400
(43) Date of publication of application: 11.04.2018
(73) Proprietor: Motus GI Medical Technologies Ltd., 3902638 Tirat HaCarmel (IL)
(72) Inventor: HASSIDOV, Noam, 2521300 Moshav Bustan HaGalil (IL); SHTUL, Boris, 2623014 Kiryat-Motzkin (IL); KOCHAVI, Eyal, 3452620 Haifa (IL); ARNON, Tzach, 2018000 Doar-Na Misgav (IL); LULEKO, Koby, 2019600 Eshchar (IL); BLECHER, Dan, 5250451 Ramat-Gan (IL)
(74) Representative: Kramer, Dani
(86) International application number: PCT/IL2016/050544
(87) International publication number: WO 2016/189533

(56) References cited:
- WO-A1-2009/095915
- WO-A1-2009/095915
- WO-A1-2015/075721
- US-A- 5 725 476
- US-A- 5 725 476
- US-A1- 2005 154 262
- US-A1- 2005 256 464
- US-A1- 2013 296 771
- US-A1- 2015 257 633

## Description

### FIELD AND BACKGROUND OF THE INVENTION

The present invention, in some embodiments thereof, relates to a tool for use with an endoscope and, more particularly, but not exclusively, to components to be inserted in a body lumen together with an endoscope and used to clean the body lumen to facilitate visualization of the lumen by means of the endoscope. For example, some embodiments may be used with a colonoscope for cleaning of a colon during colonoscopy.

During a colonoscopy, fecal matter is removed from a colon using pipes dedicated for the tasks of irrigation and evacuation. Typically, a colonoscope irrigation channel delivers fluid for loosening, dissolving, and/or fragmenting fecal material to the colon; and a working channel evacuates this material.

Self-cleaning systems have been described which add irrigation and/or evacuation channels on to a colonoscope probe; for example, in International Patent Publication Nos. WO 2009/143201, filed May 20, 2009 and WO 2010/138521, filed December 2, 2010.

PCT Publication WO 2009/095915 Al to NITSAN et al. discloses "The present invention is directed to a jet spray head unit suitable for being assembled circumferentially around an elongate medical instrument, comprising a ring-shaped element that is fitted with at least a first set of distally-directed apertures that extend from the distal face to the proximal face of said element, wherein each of said apertures is adapted such that the proximal end thereof may be connected to a source of pressurized irrigation fluid. The present invention further encompasses a method of treatment using said spray head unit." (Abstract) US Patent No. 5,725,476 to Yasui et al. discloses "A cap assembly of a front end of an inserting portion of an endoscope and a cap to be attached to the front end of the endoscope, comprising; a first engaging portion provided on the front end of the inserting portion of the endoscope; and, a second engaging portion provided on the cap being disengageably engaged with the first engaging portion of the endoscope. The front end of the inserting portion of the endoscope is provided with a projection whose diameter being smaller than a diameter of the front end, the projection being connected to the front end through a stepped portion." (Abstract)

### SUMMARY OF THE INVENTION

According to invention, there is provided a tip adaptor of a colon cleaning system for use with a colonoscope as defined by appended claim 1.

Further embodiments of the invention are defined by the dependent claims.

### BRIEF DESCRIPTION OF THE SEVERAL VIEWES OF THE DRAWINGS

Some embodiments of the invention are herein described, by way of example only, with reference to the accompanying drawings. With specific reference now to the drawings in detail, it is stressed that the particulars shown are by way of example, and for purposes of illustrative discussion of embodiments of the invention. In this regard, the description taken with the drawings makes apparent to those skilled in the art how embodiments of the invention may be practiced.

In the drawings:
FIGs. 1A-1B present simplified schematic views of a cleaning system usable with an endoscope, according to some exemplary embodiments of the present invention;
FIGs. 2A-2C schematically illustrate a tip adaptor which comprises a shield, according to some exemplary embodiments of the invention;
FIGs. 2D-2E show an encounter between a portion of an intestinal wall and vacuum pulled through an evacuation lumen of tip adaptor, according to some exemplary embodiments of the invention;
FIGs. 3A-3B show an encounter between a portion of an intestinal wall and vacuum pulled through an evacuation lumen of a tip adaptor provided with an extension, according to some exemplary embodiments of the invention;
FIGs. 4A-4B schematically illustrate a tip adaptor which comprises a shield having at least one aperture, according to some exemplary embodiments of the invention;
FIGs. 5A-5B illustrate drainage of fluid and suspended waste from an intestinal lumen, according to some exemplary embodiments of the invention;
FIGs. 6A-6B illustrate a shield in relation to an imaging aperture, according to some exemplary embodiments of the invention;
FIGs. 7A-7C schematically illustrate an example of a tip adaptor comprising a fully circumferential shield, according to some exemplary embodiments of the invention;
FIGs. 8A-8C schematically illustrate an example of a tip adaptor comprising an end-recessed shield, according to some exemplary embodiments of the invention;
FIGs. 9A-9D illustrate attachment of an adaptor tip to an endoscope distal end having variable diameter, according to some exemplary embodiments of the invention;
FIGs. 10A-10D illustrate over-the-end attachment of an adaptor tip to an endoscope distal end, according to some exemplary embodiments of the invention;
FIGs. 11A-11C schematically illustrate configurations for cleaning system tip adaptors mountable on distal portions of endoscopes, according to some exemplary embodiments of the invention;
FIG. 11D shows an over-the-end applied tip adaptor, associated with an endoscope distal portion, according to some exemplary embodiments of the invention;
FIGs. 12A-12B show frontal and side-sectional views, respectively, of a tip adaptor in association with an endoscope distal end, according to some exemplary embodiments of the invention;
FIGs. 13A-13C schematically illustrate configurations for cleaning system tip adaptors mountable on distal portions of endoscopes, according to some exemplary embodiments of the invention;
FIGs. 14A-14C demonstrate attachment of a tip adaptor to an endoscope probe comprising an expanded distal portion, according to some exemplary embodiments of the invention;
FIGs. 15A-15B schematically illustrate different circumferential extents of elastically deformable tip adaptors, according to some exemplary embodiments of the invention;
FIG. 15C shows an extension of housing area of a tip adaptor over a distal end expansion, according to some exemplary embodiments of the invention;
FIGs. 16A-16C illustrate an elastically deformable tip adaptor in three configurations relative to a distal portion of an endoscope, according to some exemplary embodiments of the invention;
FIGs. 17A-17B schematically illustrate configurations for cleaning system tip adaptors mountable on distal portions of endoscopes, according to some exemplary embodiments of the invention;
FIGs. 18A-18C schematically illustrate a tip adaptor comprising a shell and insert of different hardness, according to some exemplary embodiments of the invention;
FIGs. 19A-19C schematically illustrate a tip adaptor comprising an evacuation antechamber, according to some exemplary embodiments of the invention;
FIG. 20 schematically illustrates a tip adaptor comprising a flexible wall guard, according to some exemplary embodiments of the invention;
FIG. 21 schematically illustrates a sleeve assembly in a sleeve placement jig together with components of an irrigation system and a colonoscope, according to some exemplary embodiments of the invention;
FIG. 22 illustrates positions of a cleaning system distal region (comprising for example, an adaptor tip), in relation to an intestinal wall and a flexible wall guard; and
FIG. 23 schematically illustrates a particle entering a distal tip adaptor, according to some exemplary embodiments of the invention.

### DESCRIPTION OF SPECIFIC EMBODIMENTS OF THE INVENTION

The present invention, in some embodiments thereof, relates to a tool for use with an endoscope and, more particularly, but not exclusively, to components to be inserted in a body lumen together with an endoscope and used to clean the body lumen to facilitate visualization of the lumen by means of the endoscope. For example, some embodiments may be used with a colonoscope for cleaning of a colon during colonoscopy.

### Overview

A broad aspect of some embodiments of the invention relates to a tip adaptor for connecting a distal portion of an endoscope with a distal portion of a cleaning module for cleaning of a colon or other body lumen.

In some embodiments of the invention, a probe of the lumen-cleaning module (cleaning system) is dimensioned for reaching to the end of a colon, or to another portion of a gastrointestinal tract. In some embodiments, the lumen-cleaning module is adapted for pumping irrigation fluid to the distal end of the probe to dislodge, disaggregate, dissolve, and/or suspend fecal matter in a colon. In some embodiments, the lumen-cleaning module is adapted for evacuating loosened fecal matter from the colon through a lumen of the probe.

An aspect of some embodiments of the invention relates to a two-hardness or multiple-hardness construction of a tip adaptor, for example, such that an outer portion of the tip adaptor is relatively rigid, and an insert to said outer portion is relatively soft, and/or, for example, such that an outer portion of the tip adaptor is relatively soft, and an insert to said outer portion is relatively hard.

In some embodiments of the invention, a soft insert and hard outer portion are provided.

In some embodiments of the invention, the soft insert comprises sockets sized to receive connections to fluid transport tubes such as evacuation channels, irrigation fluid supply conduits, and/or pressure sensing tubes. In some embodiments, the soft insert serves to absorb and/or buffer movements of the fluid transport tubes, such that displacements due to tube motion relative to the tip are distributed in the body of the soft insert, rather than focused to the interface region between the fluid transport tubes and their respective sockets. In some embodiments, the hard portion of the tip adaptor is sufficiently hard to resist deformations due to direct external forces likely to be encountered during navigation of a colon. Potentially, this helps to shield the socket connections from deformations that might tend to loosen connections. Potentially, the relatively inflexible construction of the hard portion of the tip adaptor resists deformations that might tend to allow the tip to become impacted upon rather than slide over protrusions in the intestinal wall.

In some embodiments, the deformability characteristics of the soft insert comprise movement of about 0.1 mm in response to between 1-10 Newtons of force. In some embodiments, movement corresponds to about 0.1-0.25 mm, 0.15-0.2 mm, 0.25-0.5 mm, or range of movements having the same, greater, smaller, and/or intermediate bounds.

In some embodiments of the invention, a hard insert and soft outer portion are provided.

Optionally, the hard insert comprises sockets sized to receive connections to fluid transport tubes such as evacuation channels, irrigation fluid supply conduits, and/or pressure sensing tubes. Optionally, use of a relatively hard insert to a soft outer portion allows manufacturing of sockets to tight tolerances for receiving fluid transport and/or pressure sensing tubes, while still allowing a soft outer portion which mitigates potentially traumatic interactions between the device head and surrounding tissue during operation.

Potentially, use of a relatively hard insert provides dimensional stability during storage and/or in use. For example, stability of the connection of the socket to tubing is preserved by the hard insert being substantially resistant to deformation under the forces of use, and/or due to forces or creep during storage. Optionally a relatively hard insert provides dimensional stability of an aperture, for example, an aperture shaped to form and/or direct a fluid jet for cleaning. Without dimensional stability of a jetting aperture, jet force, shape, and/or direction is potentially altered during storage and/or use.

In some embodiments, a soft outer shell of the tip adaptor reduces the potential for trauma in the tissue due to interactions with the tip adaptor. For example, the surface of the tip adaptor itself has some rubbery give to it, allowing distribution of forces to potentially prevent focusing force to a small region of soft tissue. In some embodiments, one or more portions of the tip adaptor soft shell are configured to partially and elastically collapse when receiving force, providing still further give in the tip adaptor shape.

In some embodiments, apertures (such as jet and/or suction apertures) which are defined and made dimensionally stable by a hard insert portion are protected by a surrounding portion of soft shell. For example, the soft shell is provided with an aperture wide enough to avoid interference with the function (for example, shape and/or aiming) of a jet aperture, and surrounding the jet aperture to prevent direct tissue interactions with it. Optionally, a soft shell aperture is provided spaced from and in front of a hard insert-defined suction aperture, potentially protecting tissue from encountering a hard edge of the suction aperture.

In some embodiments, the deformability characteristics of the soft shell (for example, the shell where it directly overlies a hard insert portion) comprise movement of about 0.1 mm in response to between 1-10 Newtons of force. In some embodiments, movement corresponds to about 0.1-0.25 mm, 0.15-0.2 mm, 0.25-0.5 mm, or a range of movements having the same, greater, smaller, and/or intermediate bounds. In some embodiments, the soft shell comprises one or more collapsible chambers and/or hollows, and is configured to allow elastic collapse to an extent (for example, of 1 mm, 2 mm, 3 mm, 4 mm, 5 mm, or another greater or smaller extent) which is at least partially determined by the dimensions of the chamber or hollow, for example in response to between 1-10 Newtons of force. The hollow is likewise, for example, about 1 mm, 2 mm, 3mm, 4 mm, 5 mm, or another greater or smaller distance in extent in the direction of collapse. Alternatively-for example, in embodiments where the chambers are sealed-the collapse is to an intermediate range governed by the compressibility of the chamber walls and/or contents.

It is to be understood that the extent and/or contiguity of the relatively hard and relatively soft parts of the tip adaptor is optionally subject to further variations and combinations. Optionally, for example, a shell comprises both hard and soft portions. For example, corners, leading surfaces, and/or other surface of the shell are formed as soft, yielding, and/or compressible portions of the shell, while other parts of the shell (exposed as surfaces and/or supporting the compressible portions from within) are formed as relatively hard portions of the shell. Additionally or alternatively, the insert portion is formed with hard, stiff, and/or incompressible portions which provide dimensional stability of parts such as socket shapes and irrigation inlet shapes, together with, for example, soft linings of sockets (potentially to allow slight deformation upon experiencing force, but thin enough so that the functionally important dimensional stability provided by the hard portion is not compromised). Additionally or alternatively, a hard socket and/or irrigation inlet is embedded within a matrix of softer material. Potentially, this allows some give and/or relative motion of sockets to absorb forces exerted on the tip, while preserving dimensional stability needed, for example, to securely hold fittings and/or reliably shape jet formation. Thus, a tip adaptor is optionally provided with multiple layers of alternating hardness and softness, within the shell and/or within the insert, which potentially allows providing at least some of the advantages of each material type.

Furthermore, although the embodiments herein are described with respect to an optionally separable "shell" and "insert", it is to be understood that both elements are optionally manufactured as a single unit. Alternatively, either element is optionally manufactured as a plurality of parts, which optionally interconnect to one another, and/or are held to the tip adaptor by the complementary part or assembly of parts. For example, in some embodiments, an insert is optionally formed as a plurality of inserts which are separately fit into the shell. Additionally or alternatively, a shell is formed of parts which attach separately to an insert, for example, by being molded into place, by a snap fit, and/or by adhesive attachment.

Moreover, it is to be understood that the terms "soft" and "hard" as used herein relate to the relative elastic deformability of materials and/or construction (including plastic polymer compositions such as polyurethane, and/or constructions comprising hollows, thin walls, or other deformable and/or reducible shapes), and, in particular, deformability in response to compressive forces.

An aspect of some embodiments of the invention relates to structures for protecting colon tissues from accidental damage by suction used for evacuation of fecal material.

In some embodiments of the invention, an intake guard is provided as a portion of a tip adaptor. In some embodiments, the intake guard comprises a wall positioned to divert suction into the intake of an evacuation channel away from the tissue of a lumenal wall during insertion to a colon. It is a potential advantage to provide such a guard wall, as the intake aperture of the evacuation port is subject in some embodiments to suction.

Tissue approaching an unshielded evacuation port may be damaged by suction, particularly if the tissue seals across the port such that the pressure gradient drop occurs substantially across the tissue. Another potential advantage of a guard wall is to protect the function of evacuation port itself. In some embodiments, a sensor system allows a controller to reduce and/or reverse suction strength upon detection of an occlusion of the system. It is a potential advantage to reduce the occurrence of occlusions by tissue in order to maintain a higher rate of evacuation throughput by the cleaning system.

The "intake aperture" or "intake cross-section" of the evacuation lumen refers to the most distal region of the evacuation lumen where the evacuation lumen is substantially uniform in cross-section (for example, within ±10% of its diameter and/or cross-sectional area along the main body of the evacuation lumen). In embodiments where a guard wall forms the last portion of the evacuation lumen, the intake aperture or intake cross-section terminates where the guard wall shape defines (or, where change is continues, where it begins to define, for example by a slope change) a substantial change in the shape of the evacuation lumen cross-section. A "suction intake" or "suction inlet" marks a region beyond which fluid is not in the evacuation lumen at all. The region interconnecting a suction intake and an intake aperture or cross-section is defined, in some embodiments, by a guard wall. In some embodiments, the interconnecting region comprises an evacuation antechamber.

In some embodiments, the suction intake defined by the guard wall is of a larger diameter than the evacuation lumen or lumens it protects, for example, larger in area by 50%, 100%, 200%, 500%, or another intermediate, smaller, or larger difference. It is a potential advantage for the suction intake to be larger, such that it is more difficult to bring tissue close to it where the tissue experiences a high pressure gradient. In some embodiments of the invention, the suction intake is formed to a non-circular shape, such as an oval, crescent, and/or slit. In some embodiments of the invention, a shortest distance across the suction intake is smaller than the smallest dimension across an evacuation lumen, for example, 75%, 50%, 25%, or an intermediate, larger, or smaller relative size. In some embodiments, the distance of maximum distal extension of the guard wall is, for example, 3-5 mm, 4-10 mm, 6-15 mm, 10-20 mm, or another shorter or longer distance. In some embodiments, the thickness of the intake guard wall is at least 0.1-0.2 mm, 0.1-0.3 mm, 0.2-0.5 mm, 0.4-1.0 mm, or another larger or smaller thickness.

According to the embodiment, the inner diameter of an evacuation channel extended by a guard wall is, for example, 2.1 mm, 3 mm, 4 mm, 4.2 mm, 4.5 mm, 5 mm, 5.5 mm, 6 mm, another larger or smaller diameter, or any diameter in between. The number of evacuation channels which a guard wall protects may be 1, 2, 3, 4 or more evacuation channels, according to the embodiment.

It is a potential advantage for the suction intake to have such a smaller relative dimension, to help ensure than particles which pass it are small enough in at least one dimension to pass through the evacuation lumen without occluding it. It is also a potential advantage for the suction intake to have a dimension of relatively large extent (for example the length of a slit or oval, which may itself be straight and/or curved), such that it is unlikely that any single large particle could act to block it completely. In some embodiments, the ratio of longest to shortest dimension of a suction intake is, for example, 1:2, 1:3, 1:5, 1:10, or another intermediate, smaller, or larger ratio.

In some embodiments of the invention, the intake guard is adapted with one or more additional features which avoid interference with and/or support functioning of the endoscope probe and/or cleaning system. In some embodiments, the intake guard, extending distally from the endoscope probe end, is provided with a tapering shape. The intake guard, in some embodiments, is more fully circumferential (optionally, fully circumferential) than is required simply to shield the evacuation port. It is a potential advantage to provide a taper on the intake guard, for example, so that the narrower tip presented thereby can insert into (and potentially help to pry apart) intestinal constrictions during forward navigation. The wall of the intake guard tapers, for example, to the diameter of the distal end of the colonoscope probe. In some embodiments, the guard tapers more or less, for example, to within about 120% of the colonoscope probe diameter, or within about 100%, 90%, 80%, 50% or another larger or smaller relative taper diameter. In some embodiments, the tapering extent is different at different circumferential locations. For example, a taper, in some embodiments, is shorter (distally) and/or wider (radially) at a portion of the adaptor which is closer to an imaging and/or illumination means comprised within the colonoscope. Potentially, this avoids blocking the view and/or casting a visible shadow. In some embodiments, the taper begins behind the distal end of a colonoscope, and terminates at about the distal end.

In some embodiments, the intake guard is provided with one or more vent apertures that allow fluid to cross the intake guard wall, while still preventing tissue of a lumenal wall from approaching regions of high negative pressure. In some embodiments, apertures are positioned at locations on the guard wall which are sufficiently far from the region of maximal pressure drop that there is no damaging pressure drop across them. This may be, for example, near the open end of the guard wall that defines the suction aperture. Additionally or alternatively, an aperture is located or near an interior cross-section defined by the guard wall which is relatively large, such that flow is slower, and the pressure drop correspondingly less. Potentially, having a dedicated, wall- and/or debris-protected suction aperture separate from the vent apertures reduces the pressure gradient across the vent apertures to a level which is unable to grab and/or injure intestinal tissue.

A vent aperture provides a potential advantage for allowing more complete removal of fluid from a body lumen. A guard wall, for example, might otherwise act as a barrier (for example, because of an inward taper) that prevents fluid from reaching proximity to the evacuation port.

In some embodiments, an intake guard is shaped to a rounded distal surface, for example, a surface approximating the surface of a sphere, ovoid, or other substantially rounded shape having a radius of about 10-20 mm. In some embodiments, the surface curvatures approximate a radius within a range of about 5-15 mm, 10-25 mm, 15-25 mm, 20-30 mm, or another range of radii having the same, larger, smaller and/or intermediate bounds.

In some embodiments, an intake guard positions vent apertures (evacuation channel access apertures) such that an evacuation antechamber is positioned between the vent apertures and the intake apertures of the evacuation chamber itself. In some embodiments, the evacuation antechamber comprises an open region fluidly interconnecting a plurality of evacuation intake apertures proximally. In some embodiments, the evacuation antechamber comprises another aperture which is positioned in a location sheltered from waste and/or intestinal wall suction contact (for example, in fluid communication with a lumen of the tip which is difficult to block, due the size, shape and/or position of its own openings). Potentially, the sheltered aperture acts as a pressure shunt to prevent a suction gradient from increasing across one or a plurality of vent apertures if the one or plurality of vent apertures is blocked (for example, by waste particles).

In some embodiments, vent/access apertures are arranged axially in line with evacuation intake apertures, at a size about equal to or smaller than the intake apertures. Potentially, this relative positioning and relative size allows the access apertures to operate as size- and/or orientation-selective filters for waste particles entering the evacuation antechamber, such that fewer waste particles reach the relatively high suction gradient of the intake aperture that are too large and/or oriented to block it.

An aspect of some embodiments of the invention relates to variable-site positioning for a tip adaptor which mates a cleaning system probe to an endoscope probe.

In some embodiments of the invention, the tip adaptor is mateable to a distal end of an endoscope probe directly from the side. In some embodiments, this is obtained by a slit along the side of the tip adaptor which is expandable to receive the endoscope probe, and contractible to lock the tip around the endoscope probe. Optionally, the expandability and contractibility comprise elasticity of the tip adaptor materials. In some embodiments of the invention, the tip adaptor is mateable to a distal end of an endoscope probe over the distal end of the endoscope probe, and configured to be tightened around a portion of the endoscope probe proximal to the end. Potentially, one or both of these configurations allows a tip adaptor to be positioned for a desired balance between: sufficiently distal to provide colonic cleaning but sufficiently proximal to reduce interference with probe navigation in a lumen.

In some embodiments, an endoscope probe is provided with an expanded tip (for example, to accommodate structures for endoscope functions) and a cleaning system tip adaptor is attachable proximal to this expansion. It is a potential advantage to attach proximal to the region of expansion, since this allows a tip adaptor, in some embodiments, to conform to a smaller-diameter region. Conforming to a smaller-diameter region in turn potentially reduces the overall diameter added to the distal portion of the endoscope probe by the cleaning system probe.

An aspect of some embodiments of the invention relates to a flexible guard wall which acts as a variable-distance standoff between a cleaning system tip adaptor and the intestinal wall.

In some embodiments, the guard wall is positioned to stick radially away from the position of the fluid access and/or intake aperture of an evacuation channel, such that suction applied to the evacuation channel is restricted from pulling intestinal wall portions up to the aperture itself (with potentially resulting injury). In some embodiments, the wall is provided with sufficient stiffness to act as a spring that bends to convert distal-proximal motions of the colon cleaning distal end into motion across the transverse cross-section of the colon. For example, with the guard wall bent backward from the tip adaptor (bent proximally) the tip body moves toward or away from the wall as the tip is advanced distally or proximally, respectively. In some embodiments, this motion is used to aim jets, set a level of tip immersion for evacuation, and/or select a tip position for navigation of a restriction, constriction, and/or barrier to distal movement.

Before explaining at least one embodiment of the invention in detail, it is to be understood that the invention is not necessarily limited in its application to the details of construction and the arrangement of the components and/or methods set forth in the following description and/or illustrated in the drawings. The invention is capable of other embodiments or of being practiced or carried out in various ways.

For convenience of exposition, cleaning systems, modules, and/or methods described herein are sometimes referred to as "colon cleaning" systems, modules and/or methods. Colon cleaning is contemplated as a common use of embodiments of the invention. However, it is to be understood that methods and devices taught herein may also be used to clean other portions of an intestine and/or other body lumens.

Accordingly, the term "colon cleaning" as applied to these methods and devices encompasses cleaning not only the colon, but also other portions of an intestine and/or other body lumens. For example, some embodiments of the invention are potentially useful in cleaning as part of a procedure for diagnosing and treatment bleeding in the upper GI tract.

### Reference Embodiment

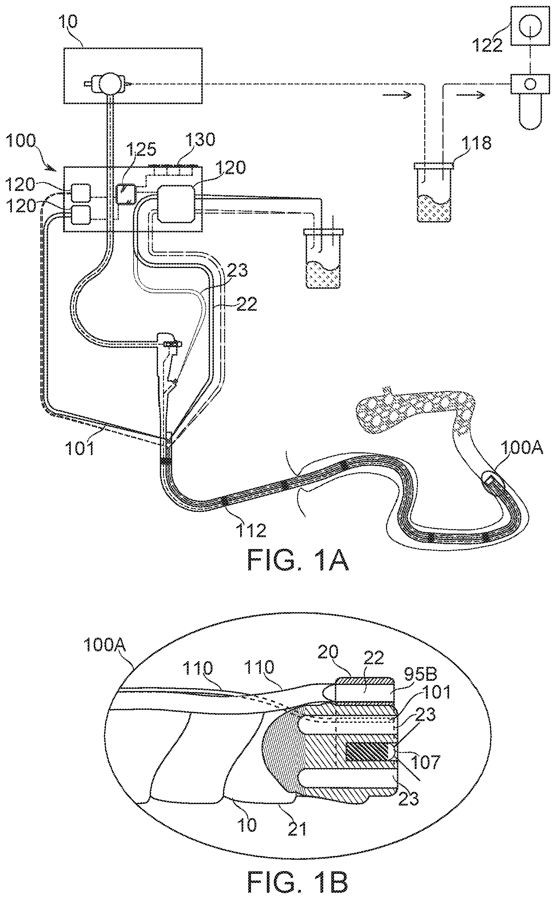

Reference is now made to *Figures 1A-1B**,* which are simplified schematic views of a cleaning system **100** usable with an endoscope **10** (which may be a colonoscope), according to some exemplary embodiments of the present invention. *Figure 1B* shows inset region **100A** of *Figure 1A* in greater detail.

In some embodiments cleaning system **100** comprises an endoscope **10,** and in some embodiments cleaning system **100** is independent of and optionally usable with an endoscope **10.** In some embodiments, system **100** may also be used independently as an insertable cleaning system not connected to or used with an endoscope.

System **100** comprises an interface device **20** which in some embodiments is designed to be disposable, i.e. for one-time use. In some embodiments interface **20** is a distal portion of cleaning system **100,** and serves to connect system **100** to a distal portion of an endoscope **10,** as shown in the figure. In some embodiments, endoscope **10** is a colonoscope. In some embodiments, endoscope **10** comprises an imaging port **107** and/or other imaging means at a distal portion thereof **21.**

Interface **20** is optionally attached to at least one and optionally a plurality of flexible tubes **110,** also optionally disposable. In some embodiments flexible tubes **110** are sufficiently long to connect interface **20** to proximal components of cleaning system **100** while interface **20** and a distal portion of endoscope **10** are advanced into a body lumen such as a colon. In an exemplary embodiment tubes **110** are 4 meters long. In other embodiments, tubes **110** are between 2 and 5 meters long. Optionally, tubes **110** are taped or otherwise temporarily attached to endoscope **10** by attachments **112** (e.g. biocompatible tape, or releasable clamps positioned at convenient intervals, for example about every 10-20 cm, every 5-35 cm, or over another longer or shorter interval). Tapes may optionally be localized tape portions, and may optionally be one or more long tape pieces spirally wrapped around an endoscope and its accompanying tubes.

In some embodiments (for example, any of the interface device embodiments described herein), the distal portion of a colonoscope probe **21** has a diameter, of, for example, 5-8 mm, 6-10 mm, 8-12 mm, 10-15 mm, 14-20 mm or another range of diameters having range boundaries equal, higher, lower, or intermediate to the ranges given. A colonoscope distal end diameter may be variable within, for example, the distal 1-10 cm of its length, due to irregularities in construction, structures for steering, and/or structures for containing colonoscope instrumentation. An interface device for connecting to the distal end of a colonoscope probe extends, for example, 1-2 mm, 2-5 mm, 4-8 mm, 6-12 mm, 10-20 mm, 10-30 mm, or another range of distances having bounds equal, intermediate, smaller, or larger.

In some embodiments, tubes **110** are positioned around endoscope **10,** may wrap around endoscope **10** and/or around each other, and in general are positioned with respect to endoscope **10** according to convenience and/or in a manner which enhances simplicity of operation and/or flexibility of body-insertable portions of system **100** together with endoscope **10.**

In some embodiments, tubes **110** comprise an evacuation channel **22** which connects interface device **20** to a vacuum source, which may for example be a pump **120** and/or a connection to a centralized vacuum system **122** such as are available in some hospitals and clinics. In some embodiments, system **100** comprises a plurality of evacuation channels **22** (also called "suction tubes **22"** herein), such as for example two, or three, or four, or more tubes **22.** In some embodiments, a lumen of an endoscope working channel **23** is usable as an evacuation channel. In some embodiments, a pump **120** is reversible to purge an evacuation channel. According to the embodiment, the inner diameter of an evacuation channel is, for example, 2.1 mm, 3 mm, 4 mm, 4.2 mm, 4.5 mm, 5 mm, 5.5 mm, 6 mm, another larger or smaller diameter, or any diameter in between.

In some embodiments, interface device **20** (and/or any of the other interface devices described herein) comprises structure which adapts from the intake aperture of an evacuation channel **22** to another shape at the distal-most portion of the suction intake, for example, a slit, an oval, an annulus, and/or a partial annulus. In some embodiments, two or more evacuation channels are combined into a single suction inlet by the interface device. In some embodiments, a single evacuation channel is split into two or more separate suction inlets. In some embodiments, the area of the suction inlet aperture is larger than the cross-section of the evacuation channel lumen, for example, 50% larger, 100%, 150%, 300%, 500%, or another intermediate, larger or smaller difference in area.

In some embodiments one or more or tubes **110** serves as an irrigation tube **101,** connected to a fluid source which is a source of water or another liquid and/or a liquid/gas combination. In some embodiments, one or more working channels of endoscope **10** serves as irrigation tube **101.**

In some methods of use, irrigation tube **101** supplies a cleaning fluid to interface device **20,** which delivers it into a body lumen such as a colon, optionally under pressure, where it serves to wash the colon, and loosens and partially dissolves fecal matter which is then suctioned into one or more suction portals (also called "suction inlets" herein) **95B** in interface **20** (as shown in figures discussed below), and thence into evacuation channel **22** and/or **23,** which transport the material out of the body and deliver it to an optional fecal matter collector **118.** Vacuum (i.e. suction) in tubes is optionally regulated by a valve or regulator or variable pump **120,** optionally connected to vacuum source **122.** Delivery of suction to tube(s) **22** and of irrigation fluid to tube(s) **101** is optionally regulated by a controller **125,** optionally receiving commands from an operator through a user interface **130** such as a computer console and/or a knob or lever or other manual command.

### Vacuum Shield

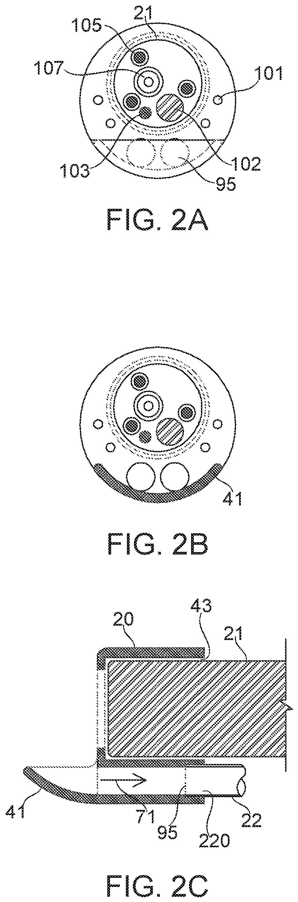

Reference is now made to *Figures 2A-2C**,* which schematically illustrate a tip adaptor **20** which comprises the wall of an intake guard **41,** according to some exemplary embodiments of the invention.

For some embodiments of the invention, *Figures 2C* and *2A**-2B* present side cross-sectional and end cross-sectional views at different depths respectively of a tip adaptor **20** comprising an intake guard **41** designed to distance body tissue from an evacuation portal **95** which might be blocked by body tissue and/or which might damage body tissue.

In some embodiments, intake guard **41** is positioned between an evacuation portal such as portal **95** and tissues of a body when said tip adaptor is inserted in a body lumen. For example, intake guard **41** protects tissues of the walls of a colon when suction is applied to the colon through evacuation portal **95.** In some embodiments, intake guard **41** extends along at least 20% of the circumference of tip adaptor **20,** extends distally from tip adaptor **20.** In some embodiments, intake guard **41** extends along at least 35%, 50%, 80%, or 100% of the circumference of tip adaptor **20,** or a lesser fraction of the circumference, or any intermediate circumference fraction. In some embodiments, the distance of maximum extension of intake guard **41** is, for example, 3-5 mm, 4-10 mm, 6-15 mm, 10-20 mm, or another shorter or longer distance. The distance of shortest extension at any given point around its circumference, in some embodiments, is between 0 mm and any distance up to the longest distance of extension. In some embodiments, the thickness of intake guard wall **41** is at least 0.1-0.2 mm, 0.1-0.3 mm, 0.2-0.5 mm, 0.4-1.0 mm, or another larger or smaller thickness. In some embodiments, intake guard **41** is rigid or semi-rigid, such that it maintains its shape under at least the pressure used to normally advance the endoscope probe into the body lumen. A rigid or semi-rigid intake guard **41** potentially assists in forward penetration of an advancing distal end of an endoscope probe. In some embodiments, intake guard **41** is flexible, sufficiently so that it collapses, for example, when pressed directly to a lumenal wall. This is a potential advantage for reducing injury as an endoscope probe advances. In some embodiments, intake guard **41** is designed with a flexibility/rigidity which comprises both navigation assistance and safety collapse, such that it collapses, for example, under an advancing pressure of 1-4 PSI, 3-8 PSI, 5-10 PSI, 8-15 PSI, or another higher or lower threshold of pressure. Intake Guard **41** is an optional feature of any of the cleaning systems and/or tip adaptors described herein.

Protection offered by intake guard **41** is potentially by one or more of a selection of mechanisms, as now described, and/or by another mechanism. In some embodiments, shield **41** positions a suction inlet which is in fluid communication with an evacuation lumen **22** to a location and/or orientation which is less exposed to wall contacts than the bare intake aperture **95** of the evacuation lumen **22.**

Optionally, the position is a more medial position, potentially increasing a distance between a body lumen wall and a region of a high pressure gradient. Optionally, the orientation is shifted to make contacts with the wall less likely, for example, a rotation which is about mid-way between an orientation perpendicular to the distal-proximal axis of the device (which may tend to come into contact with wall protrusions during distal motion) and an orientation parallel to the distal-proximal axis (which may tend to come into contact with flat regions of the wall). Mid-way, in some embodiments of the invention, comprises an angle which is at least 30° degrees away from both axes, or at least 35°, 40°, or another intermediate, larger, or smaller angle of separation. In some embodiments, the aperture is both moved and rotated. For example, the suction inlet illustrated in *Figures 2A-2C**,* defined by the protruding wall section of intake guard **41,** rotates the suction inlet by about 90° to point in a medial direction, and moves the aperture in a medial direction.

In some embodiments of the invention, intake guard **41** protects by adopting a shape which is unlikely to be matched by an occluding section of wall. For example, the lip of the intake guard may be formed to define non-planar aperture mouth (for example, the margin of the intake guard forms a lip which is not substantially contained within a single plane), and/or the aperture mouth may be guarded on one or more sides by structures that prevent a wall section from overlying the aperture. For example, the distal end of the colonoscope itself prevents the aperture of intake guard **41** in *Figure 2C* from being fully occluded by a large extent of intestinal wall. In some embodiments, interference in the region of the suction inlet mouth prevents fully occlusion by an extent of wall which is continuous beyond 10 mm from the point of contact, or beyond 5-10 mm, 10-15 mm, 8-20 mm, or another range of continuous extent having bounds which are the same, intermediate, smaller, or larger.

In some embodiments of the invention, intake guard **41** protects by creating a larger suction inlet area, decreasing the likelihood of total occlusion. For example, irregularities **1A** of an intestinal wall are limited in size, so a sufficiently large aperture, even if oriented to the surface of the irregularity, is not occluded. In some embodiments of the invention, the suction inlet is large enough to prevent total occlusion by a flap of tissue which is less than 5 mm in protruding extent. In some embodiments, occlusion is prevented for flaps of tissue having protruding extent less than 5-10 mm, 2-5 mm, 8-12 mm, or another range of extents having bounds equal, intermediate, larger, or smaller.

Another potential advantage of a large suction inlet area is to help ensure that regions of high pressure differential with the intracolonic pressure are located deep within the intake structures of the evacuation channel and/or intake guard, rather than exposed near the intake aperture itself.

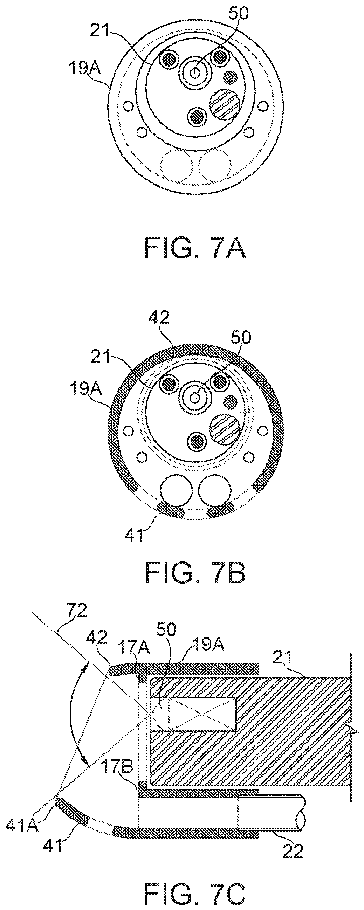

Reference is now made to *Figures 7A-7C**,* which schematically illustrate an example of a tip adaptor **19A** comprising a fully circumferential intake guard **41,** according to some exemplary embodiments of the invention. *Figures 7A-7B* show frontal views at different section planes. *Figure 7C* shows a view in horizontal cross-section.

In some embodiments, intake guard portion **42** protrudes a shorter distance away from the tip than a region **41A** protecting evacuation lumen **22.** A potential advantage of a fully circumferential intake guard **41** is to provide a narrower entry point for an advancing into a body lumen.

In some embodiments, the shorter region **42** is positionable to be radially nearer to an imaging device **50,** to reduce or prevent obstruction of a field of view **72** of imaging device **50** by the extension of intake guard **41.** In some embodiments, one or more positioning elements **17A, 17B** act to ensure proper relative positioning of tip adaptor **19A** relative to the end of distal endoscope region **21.**

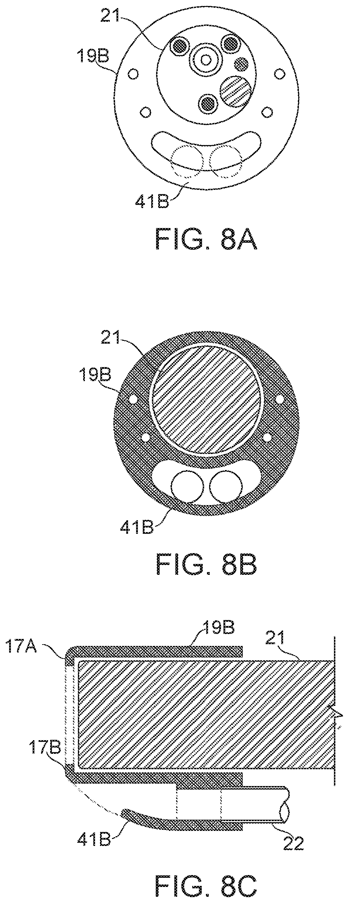

Reference is now made to *Figures 8A-8C**,* which schematically illustrate an example of a tip adaptor **19B** comprising an end-recessed intake guard **41B,** according to some exemplary embodiments of the invention. *Figures 8A-8B* show frontal views at different section planes. *Figure 8C* shows a view in horizontal cross-section.

In some embodiments, intake guard **41B** is recessed away from the distal end of tip adaptor **19B.** A potential advantage of this position is to protect of an evacuation lumen **22,** with lowered obstruction of the progress and/or field of view of distal endoscope region **21.** In particular, the extreme distal end of the advancing distal end is expanded only by the thickness of tip adaptor **19A** used to house the anchoring structures of the adaptor. In some embodiments, one or more positioning elements **17A, 17B** act to ensure proper relative positioning of tip adaptor **19A** relative to the end of distal endoscope region **21.**

Referring again to *Figures 2A-2C**,* chamber **43** of tip adaptor **20** is for housing a distal end of an endoscope **21.** A chamber **220** in the figure is for housing and/or connecting to (and functionally extending) a tube **22** which optionally connects proximally to a vacuum source as described above. Suction **71** produced by a proximal vacuum source is delivered in some embodiments by tube **22** and chamber **220** to an evacuation portal **95.**

In some embodiments of the invention, tip adaptor **20** comprises a distal intake guard **41.** Potentially, intake guard **41** serves to separate body lumen wall tissue or other body tissue from direct exposure to high levels of vacuum potentially present in an evacuation portal **95.**

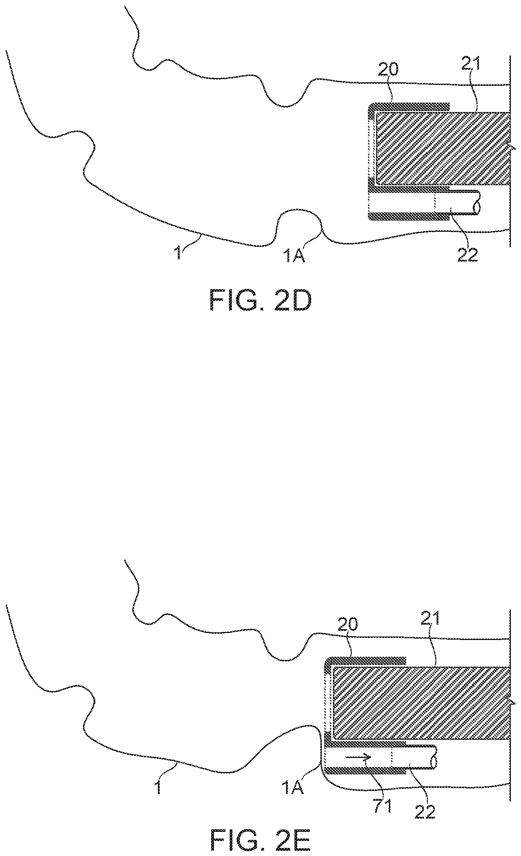

Reference is now made to *Figures 2D* and *2E*, which show an encounter between a portion **1A** of an intestinal wall **1** and vacuum pulled in direction **71** through an evacuation lumen **22** of tip adaptor **20,** according to some exemplary embodiments of the invention. In *Figure 2D**,* an advancing distal end of an endoscope **21** approaches an irregularity **1A** in wall **1.** In *Figure 2E**,* irregularity **1A** is pulled to the aperture of evacuation lumen **22.** It is, however, a potential advantage to prevent this mode of contact between an active evacuation portal and soft body tissue. Faults which potentially occur in this scenario include:
- body tissues sucked towards and into the evacuation portal blocking the portal, preventing proper functioning of a lumen-cleaning process; and
- traumatizing of body tissues sucked into the evacuation portal.

Suction strong enough to provide efficient and rapid evacuation of fecal matter from a colon (for example) is potentially strong enough to create hematoma, ruptured blood vessels, or other undesirable outcomes in tissue subject to a strong suction.

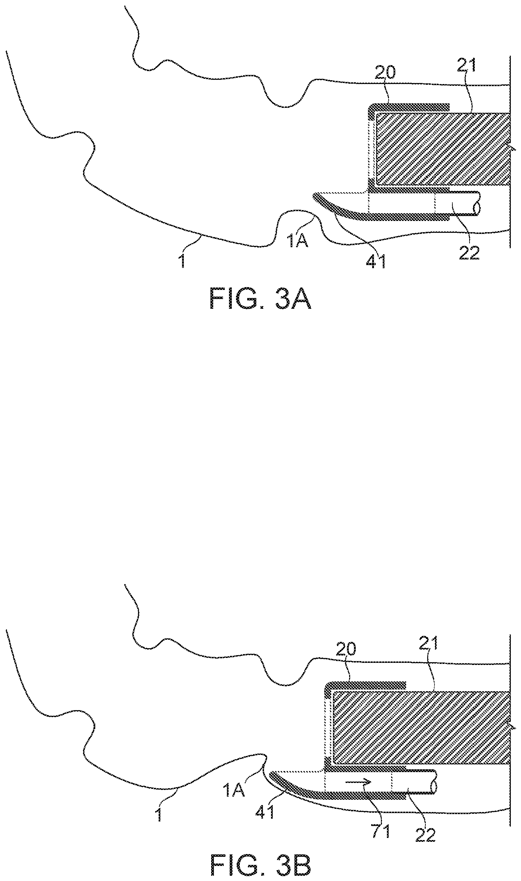

Reference is now made to *Figures 3A* and *3B*, which show an encounter between a portion **1A** of an intestinal wall **1** and vacuum pulled in direction **71** through an evacuation lumen **22** of tip adaptor **20,** provided with an extension **41,** according to some exemplary embodiments of the invention. In *Figure 3A**,* an advancing distal end of an endoscope **21** approaches an irregularity **1A** in wall **1.** In *Figure 3B**,* irregularity **1A** is pushed forward, away from the aperture of evacuation lumen **22,** protecting it from suction force **71.**

Returning now to *Figures 2A-2C**,* in some embodiments, extension **41** stands between evacuation portal **95** and sensitive tissue such as the wall of a colon, and protects that tissue from damage while preventing blockage of evacuation portal **95** by body tissue. In some embodiments, intake guard **41** is positioned near evacuation portal **95.**

Optionally a plurality of shields **41** is positioned near a plurality of evacuation portals **95.** Optionally, intake guard **41** is positioned around all or major portions of tip adaptor **20** and not be limited to positions in direct proximity to evacuation portals **95.**

Optionally, intake guard **41** is curved towards a central axis of tip adaptor **20,** as shown in *Figure 2C**.*

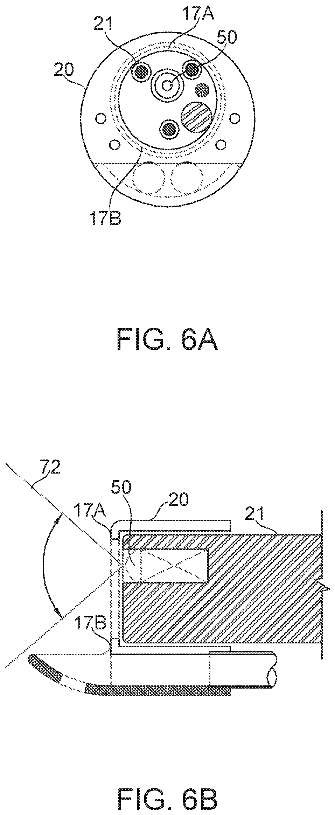

Reference is now made to *Figures 6A-6B**,* which illustrate an intake guard **41** in relation to an imaging aperture **50,** according to some exemplary embodiments of the invention.

Optionally, intake guard **41** is positioned to avoid or partially avoid limiting the field of view **72** of optical components of endoscope **10.**

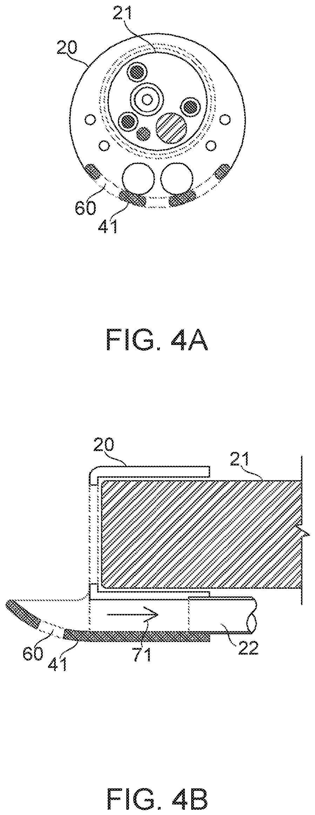

Reference is now made to *Figures 4A-4B**,* which schematically illustrate a tip adaptor **20** which comprises an intake guard **41** having at least one aperture **60,** according to some exemplary embodiments of the invention.

In some embodiments of the invention, intake guard **41** may comprise one or more (optionally small) holes **60** through which cleaning can occur. This configuration is shown in side cross-section in *Figure 4B* and in front cross-section in *Figure 4A**.*

Optionally, holes **60** may have rounded edges so that they will not cut or scrape tissues of the lumen walls as tip adaptor **20** advances in the body lumen.

The appropriate size and location of holes **60** may depend on the characteristics of the material to be cleaned, the nature of the irrigation used, the strength of the suction provided, and various other operational parameters. *Figures 4A* and *4B* present exemplary and non-limiting embodiments. In some embodiments, holes **60** are placed such that they overlie interior regions exposed to suction which experience a relatively small pressure differential with the intracolonic pressure. For example the holes may be placed near the distal end of an intake guard wall **41,** and/or may be placed such that the nearest portion of the chamber formed on the interior side of the intake guard wall **41** is relatively large.

In some embodiments, this larger chamber region corresponds to an effectively increased "pipe" diameter, resulting in a lower pressure drop, for example according to Bernoulli's principle. This is a potential advantage for the safety of the tissue, and/or for reduction of the tendency of suction to grab approaching tissue.

In some embodiments, a potential tension exists within the goal of evacuating as much waste volume as practical, while exposing the tissues of the colonic segment to a pressure gradient which is as gentle as practical. In embodiments of this compromise, suction as such is not relied upon to dislodge particles. It is also a potential advantage to put apertures leading to the evacuation channel where they are exposed to the least pressure differential. It is another potential advantage for structures leading to the evacuation channel to have wide cross sections near such apertures, narrowing as they recede from regions which might be exposed to tissue.

According to some optional methods of use, a physician turns on suction in tube **22** when hole(s) **60** are appropriately positioned over an area needing cleaning, and turns off or reduces suction when holes **60** are positioned over vulnerable tissue.

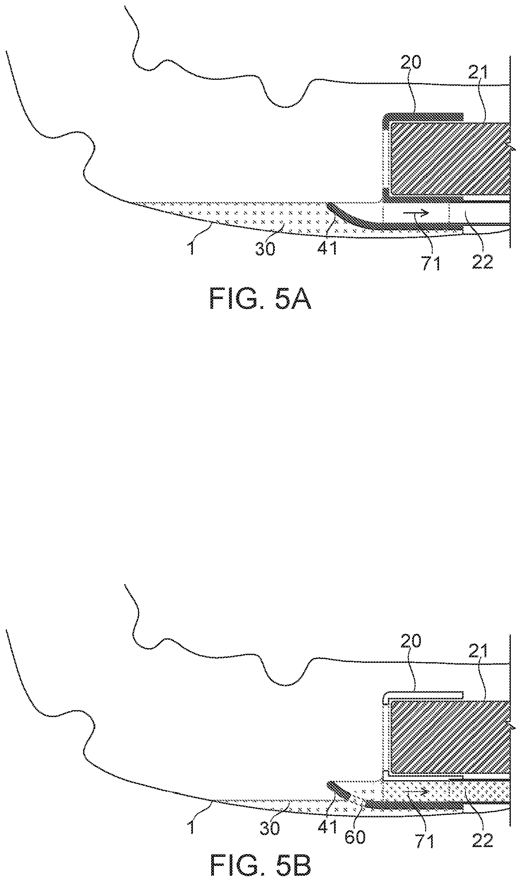

Reference is now made to *Figures 5A* and *5B**,* which illustrate drainage of fluid and suspended waste **30** from an intestinal lumen, according to some exemplary embodiments of the invention. In each figure, the lumen contained by intestinal wall **1** is being drained by vacuum pulled in direction **71** through an evacuation lumen **22** of tip adaptor **20,** provided with an extension **41.**

In *Figure 5A**,* a distal end of an endoscope **21** is partially submerged in fluid and suspended waste **30.** Suction **71** is unable to completely drain waste suspension **30,** due to the barrier comprising extension **41.** In *Figure 5A**,* a distal end of an endoscope **21** is partially submerged in fluid and suspended waste **30.** Suction **71** is provided access to remnants of drained waste suspension **30,** by one or more holes **60** provided in extension **41.**

Note that tip adaptor **20,** in some embodiments, is configured to remain clear of colonoscope elements located at the colonoscope tip, comprising, for example (as shown in *Figure 2A*), illumination LED **105,** imaging port **107,** working channel **102,** and irrigation channel **103.**

### Exemplary Tip Adaptors

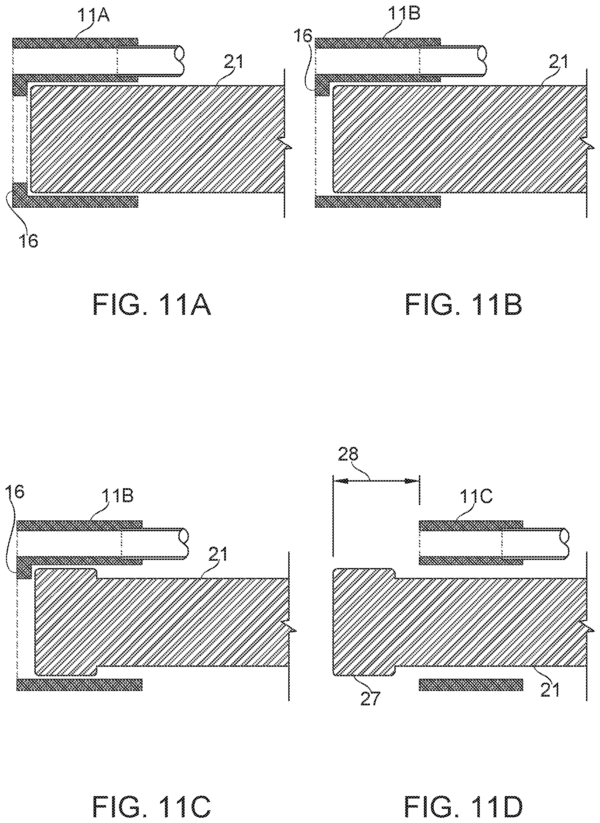

Reference is now made to *Figures 11A-11D*, 13A-13C, and *17A-17B,* which schematically illustrate configurations for cleaning system **100** tip adaptors **20** mountable on distal portions **21** of endoscopes **10,** according to some exemplary embodiments of the invention.

It is to be understood that, in some embodiments "tip adaptors" shown in these figures and referenced elsewhere herein are connectable to tubes **110** and to other portions of a cleaning system such as the cleaning system **100** of *Figures 1A-1B**.*

It is also to be understood that configurations of what is generally referred to as "tip adaptor **20"** herein should be understood to include (changed as necessary) all individually described tip adaptors described herein (for example, tip adaptors **11A-11C, 12, 13,** and **19A-19B**), and all other devices conforming to descriptions of "tip adaptors" presented herein as embodiments of the invention.

*Figures 11A-11D* schematically illustrate sectional side views of tip adaptors **11A-11C** (and **20**) mounted on a distal end of an endoscope **10.**

In exemplary embodiments shown in *Figures 11A-11C**,* tip adaptor **11A, 11B** is shaped to fit the distal end of the endoscope, optionally with a fully or partially circumferential projection **16** or other feature which assures that tip adaptor **11A, 11B** will be positioned at the distal end of endoscope **21** in a fixed positional relationship.

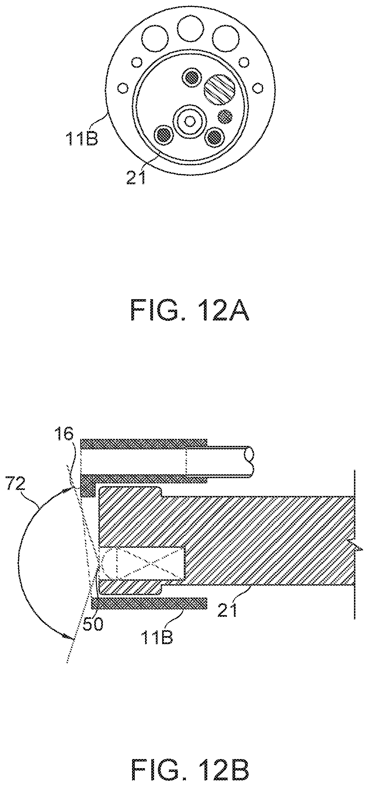

Reference is now made to *Figures 12A-12B**,* which show frontal and side-sectional views, respectively, of a tip adaptor **11B** in association with an endoscope distal end **21,** according to some exemplary embodiments of the invention.

It is a potential advantage to adjust the shape of projection **16** to accommodate the function of features located at the end of distal endoscope portion **21.** In some embodiments, for example, projection **16** may be retracted from portions of the tip adaptor **11B** which are positionable in proximity to the field of view **72** of an imaging device **50.** This retraction allows the positioning function to operate without impairing the functional operation of the endoscope optics and/or other features such as irrigation, working channels, and/or illumination.

*Figures 11D*, 13A-13C, and *17A-17B* are simplified schematics of tip adaptors which are either variably positionable on distal portions of endoscopes **21,** or which are fixedly positioned near but not at a distal end of an endoscope **21,** according to some embodiments of the invention.

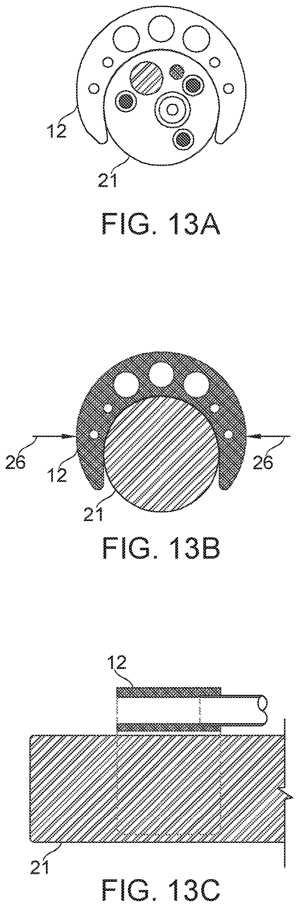

Figures 13A-C show front and front- and side- sectional views of tip adaptor **12** (which is a tip adaptor **20**) configured to be fittingly applied over a distal portion of an endoscope **21** from a lateral direction. In some embodiments, interface tip adaptor **12** is elastically deformable, and sized to grip endoscope end **21** in a pressure grip (direction of grip pressure is indicated, for example, by arrows **26**). Optionally, tip adaptor **12** is fitted by a user to a selectable position at or near the end of distal portion of endoscope **21.** In some embodiments, tip adaptor **12,** is optionally slideable for positioning along the distal body of endoscope **21.** In some embodiments, tip adaptor **12** grips endoscope distal end **21** with a sufficient strength to resist movement on endoscope distal end **21** under influence of pressures normally exerted on endoscope **21** during insertion in a colon or other body lumen.

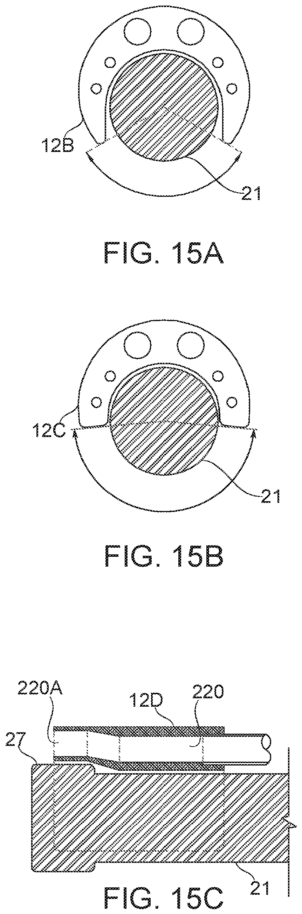

Reference is now made to *Figures 15A-15B**,* which schematically illustrate different circumferential extents of elastically deformable tip adaptors **12B, 12C,** according to some exemplary embodiments of the invention.

In some embodiments, the circumferential extent of a tip adaptor **12B, 12C** is any angle sufficient to allow a grip to be established from behind the endoscope distal portion **21** to which the tip adaptor attaches. In some embodiments, the angle is also chosen so that the adaptor can be opened wide enough to pass over the endoscope portion **21** from the side.

In some embodiments, for example, tip adaptor **12B** (*Figure 15A*) extends around about 2/3 of the circumference of the endoscope. It should be understood that the extent of circumferential wrapping is variable, depending on the diameter of the endoscope at the point of attachment.

In some embodiments, for example, tip adaptor **12C** (*Figure 15B*), extends only a small amount past 180° of the endoscope circumference at the point of attachment. In some embodiments, the security of attachment is increased by decreasing the flexibility of the tip adaptor **12C,** such that the pressing to the underlying endoscope is harder.

Thus, in some embodiments, tip adaptors which self-attach by means of their own elastic restoring force may be constructed with a relaxed-state aperture which is anywhere in size from 0% of the endoscope diameter (slit-like) to a value just sufficiently small enough allow establishing a grip. The relaxed-state aperture size may thus be, for example, 20%-80% of the endoscope diameter, or another larger or smaller size. In some embodiments, the adaptor extends from fitting about 50% around the endoscope probe profile to any larger value. A more completely enclosing adaptor potentially provides a firmer grip, with a trade-off for this comprising a larger increase in endoscope probe diameter.

The material of the tip adaptor body (for adaptors **12B, 12C,** or any other tip adaptor described herein) can be a silicone rubber or another polymer material, for example, a polymer resin otherwise used in manufacture of a colonoscope or colon cleaning system insertion tube. Exemplary Shore A durometer values for the material of a tip adaptor are between 50-70 Shore A durometer units. In some embodiments, a Shore A durometer range of 50-55 Shore A, 50-60 Shore A, 55-65 Shore A, 70-80 Shore A, 70-90 Shore A, or another range of durometer having bounds equal, intermediate, higher or lower is used.

In some embodiments, a transparent material is used to form a tip adaptor (for example, tip adaptor **12B, 12C,** or any other tip adaptor described herein). A potential advantage of transparency is more direct determination of the positioning of the adaptor on the colonoscope end.

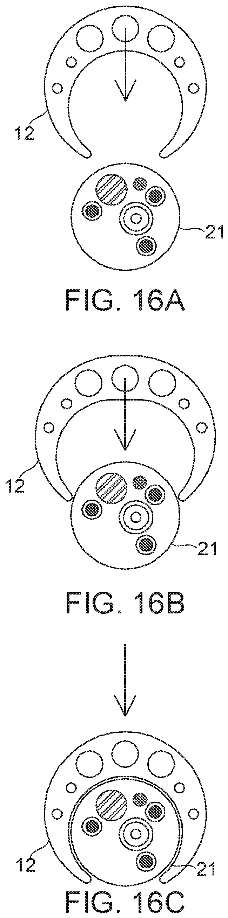

Reference is now made to *Figures 16A-16C**,* which illustrate an elastically deformable tip adaptor **12** in three configurations relative to a distal portion of an endoscope **21,** according to some exemplary embodiments of the invention.

Configurations shown are pre attachment (*Figure 16A*), mid-attachment (*Figure 16B*), and after attachment (*Figure 16C*). The sequence illustrates the flexibility of adaptor **12** for insertion over an endoscope from the side, and its capability for attachment by shape restoration once pressed onto an endoscope tube.

In some embodiments, a position for tip adaptor **20** with respect to a distal end of endoscope **21** is selected by a user according to preference and/or according to a given clinical situation, and tip adaptor **20** attached to endoscope distal end **21** according to the position selected. In some embodiments, tip adaptor **20** and/or attached tubes **110** are disposable objects intended for one-time use.

In some embodiments, distal interface **20** is fixedly connected to an endoscope distal region **21** at a position proximally spaced from the end itself (*Figure 13C*). The distance is at least, for example, 0.5-1.5 cm, 1-2.5 cm, 2-5 cm, or a shorter or longer distance from the distal end of the endoscope.

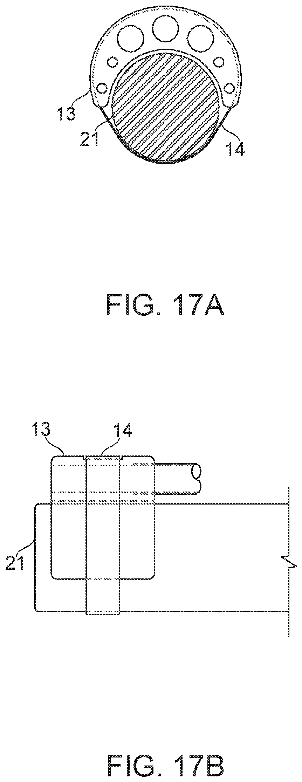

A potential advantage of the configurations shown in *Figures 13A-13C* and *17A-17B* is ensuring the optics of endoscope **10** a full field of view. If tip adaptor **20** is positioned near the end of endoscope **21** but is somewhat distanced from that end, then even unusually large tip adaptors may not interfere with full field of view for the endoscope optics.

In some methods of use in some embodiments, an endoscope **10** with attached cleaning system **100** is advanceable throughout a length of a colon with the cleaning system in use during the advance, so that the cleaning system cleans fecal matter from the length of colon. Optionally, the endoscope is subsequently gradually retracted through the cleaned colon for inspection and/or treatment. Under this method, the fact that tip adaptor **20** is positioned proximally to the distal end of the endoscope is convenient both during advancing/cleaning and during retracting/observing. During advancing, potentially, the distal tip is more easily able to penetrate restricted spaces, in virtue of not having to support the added bulk of the adaptor tip at the point of penetration. During retraction, potentially, the adaptor tip is clear of interfering with illumination and/or imaging elements in virtue of being physically out of their field of illumination and/or view.

*Figures 17A-17B* exemplify (in frontal and side views) a tip adaptor **13** which mounts on an endoscope **21,** optionally from the side of the endoscope, and is in contact with or in proximity to a distal portion of endoscope **21** along a portion (for example, about 40% or about 50% or about 60% or about 70% or about 80%) of the endoscope circumference. In some embodiments, it does not surround as much as 50% of its circumference. In some embodiments, interface tip adaptor **13** is attachable at a variety of positions along a distal portion of endoscope **21.** Optionally, tip adaptor **13** is held in place by an attachment **14** such as a bonding tape, clamp and/or any other mechanical, bonding, and/or gluing means; a strap, and/or by attachments **112** along connecting tubes **110,** themselves connected to tip adaptor **13** along the length of endoscope **10** as described above with reference to *Figures 1A-1B**.*

In some embodiments, interface tip adaptors **12** and **13** are positionable anywhere on the distal portion of endoscope **21,** to suit a physician's convenience and/or to suit the clinical requirements of a particular case. It is a potential advantage of tip adaptors having flexible and/or side-attachable, tip adaptor **13** that they are attachable for use with endoscopes of a variety of shapes and/or diameters. In some embodiments, tip adaptor **13** is constructed of elastically flexible material to further make it adaptable to endoscopes of varying sizes. For similar reasons, tip adaptor **13** is optionally adaptable to being mounted on a portion of distal endoscope **21** having a non-circular or asymmetrical cross-section.

Embodiments shown in *Figures 11D*, 13A-13C, and *17A-17B* are optionally usable to position distal outlets of "cleaning tubes" (that is, one or more irrigation tubes and/or one or more evacuation tubes) at a user-selected position with respect to a distal end of an endoscope.

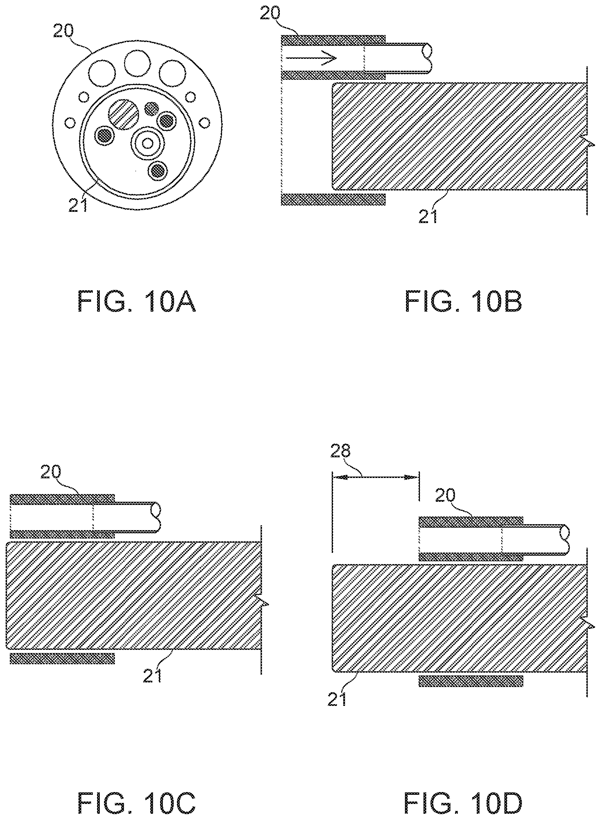

Reference is now made to *Figures 10A-10D**,* which illustrate over-the-end attachment of an adaptor tip **20** to an endoscope distal end **21,** according to some exemplary embodiments of the invention.

In some embodiments, tip adaptor **20** is attachable over the end of endoscope distal region **21** (*Figure 10B*) to a range of positions relative to the distal end. The position may be flush or nearly flush with the end (*Figure 10C*), or it may be offset proximally by a distance **28,** for example, 0.5-1.5 cm, 1-2 cm, 1.5-3 cm, or another longer or shorter offset length.

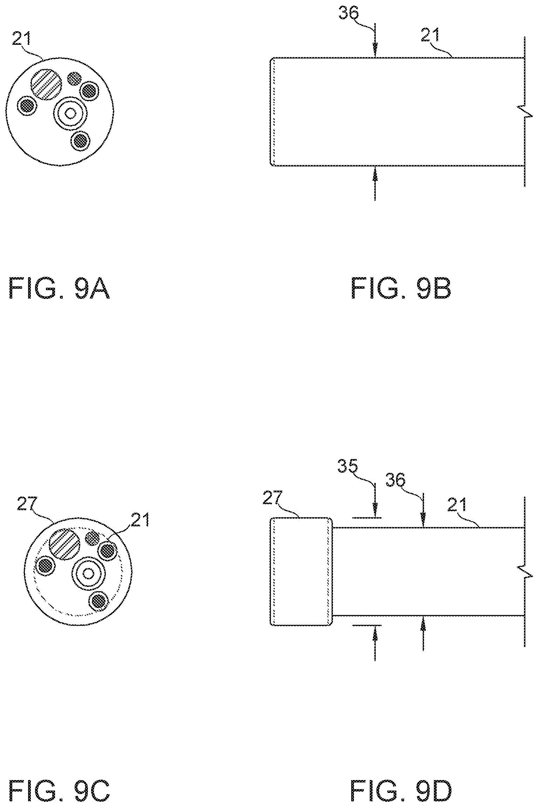

Reference is now made to *Figures 9A-9D**,* which illustrate a problem related to attachment of an adaptor tip **20** to an endoscope distal end **21** having variable diameter, according to some exemplary embodiments of the invention.

In some embodiments, an endoscope distal portion **21** comprises a relatively uniform diameter **36** extending proximally from the endoscope distal end (*Figures 9A-**9B*). In some embodiments of the invention, the endoscope distal portion is not of uniform diameter, as shown here, but may comprise irregularities, for example, due to endoscope wrapping, steering mechanism, and/or other structures. The segmented appearance of endoscope distal portion **21** in *Figures 1A-1B* reflects these irregularities. It should be noted that it is a potential advantage of elastically deformable embodiments, for example, those of *Figures 13A-13C**,* that they potentially adjust to such irregularities automatically.

In some embodiments, the irregularity is sufficiently large as to pose an obstacle to fitting an adaptor tip **20** over the distal portion. For example, the diameter **35** of expanded region **27** (*Figures 9C*-*9D*) is larger than diameter **36,** so that an over-the-end, form-fitting attachment proximal to the distal end of endoscope distal region **21** is impeded.

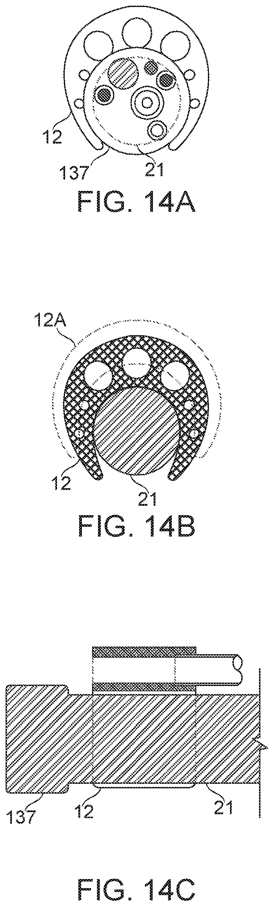

Reference is now made to *Figures 14A-14C**,* which demonstrate attachment of a tip adaptor **12** to an endoscope probe comprising an expanded distal portion **27,** according to some exemplary embodiments of the invention.

In some embodiments, endoscope distal region **21** is constructed with an expanded region **27,** for example a ring **137** encircling the distal end. Ring **137** or other expanded region **27** may be provided to fulfill functional needs of endoscopes **21,** for example to accommodate manipulating tools, illumination, and/or imaging optics. Nevertheless, such expansions potentially interfere with the navigability of an endoscope end. While sufficient care may have been taken in the design of the original endoscope to preserve maneuverability, adding a tip adaptor **20** that further enlarges the distal end of an endoscope **21** is potentially detrimental to successful navigation through a colon.

A tip adaptor such as **12** or **13** attachable to an endoscope **21** at a position proximal to an expanded distal potentially allows a narrower profile of the tip adaptor and endoscope combination. Potentially, a narrower profile is more navigable. In *Figures 14A-14C**,* for example, a tip adaptor **12** is shown attached at a region proximal to an expansion **27,** permitting a profile which is within a profile **12A** which the same adaptor would assume if placed directly over the expansion **27** of the endoscope distal end **21.**

Reference is now made to *Figure 15C**,* which shows an extension **220A** of housing area **220** of a tip adaptor **12D** over distal end expansion **27,** according to some exemplary embodiments of the invention.

In some embodiments, tip adaptor **12D** is configured to attach distally to the end of distal endoscope region **21,** with a short extension **220A,** configured to rise over an expanded region **27** and project forward, bringing an aperture of an evacuation channel **95,** for example, further forward while the main body of the tip adaptor **12** remains at a more sheltered proximal position.

Reference is now made to *Figure 11D**,* which shows an over-the-end applied tip adaptor **11C,** associated with an endoscope distal portion **21,** according to some exemplary embodiments of the invention.

A potential advantage of side-mounting tip adaptors is seen by comparison of *Figure 11D* with *Figure 14C**.* In *Figure 11D**,* the adaptor **11C** is separated by a distance **28** from the distal end of endoscope **21,** after sliding over a distal expansion **27.** However, the resulting configuration is not well fitted, and has excessive diameter relative to the diameter achievable with a configuration such as that of *Figure 14C**.* Even in the case of an over-the-end configuration mounted more securely at the distal end, as in *Figure 11C**,* the tip adaptor **11B** is potentially more obstructive than the configuration of *Figure 14C**.*

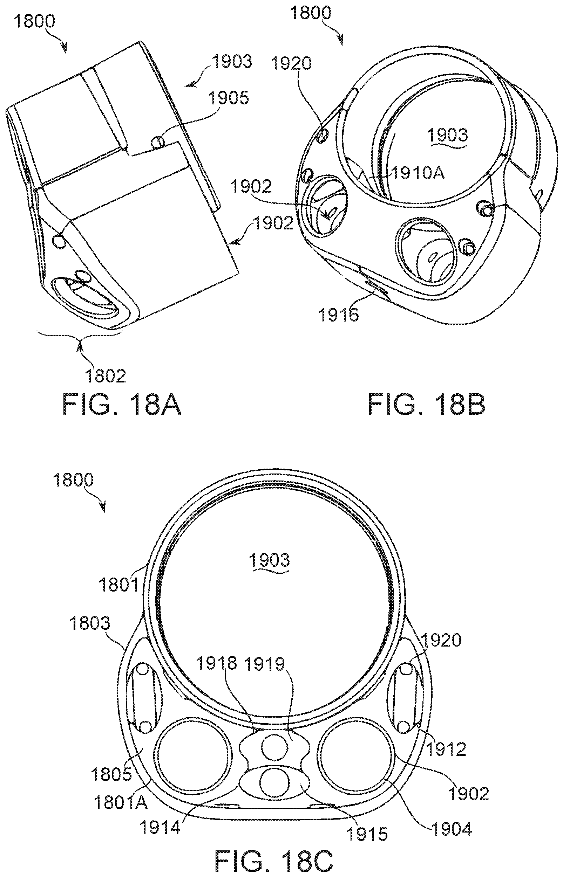

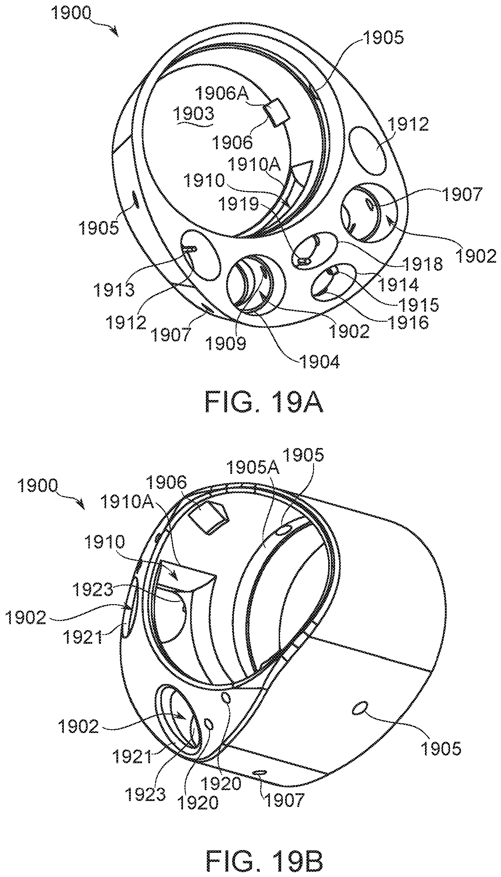

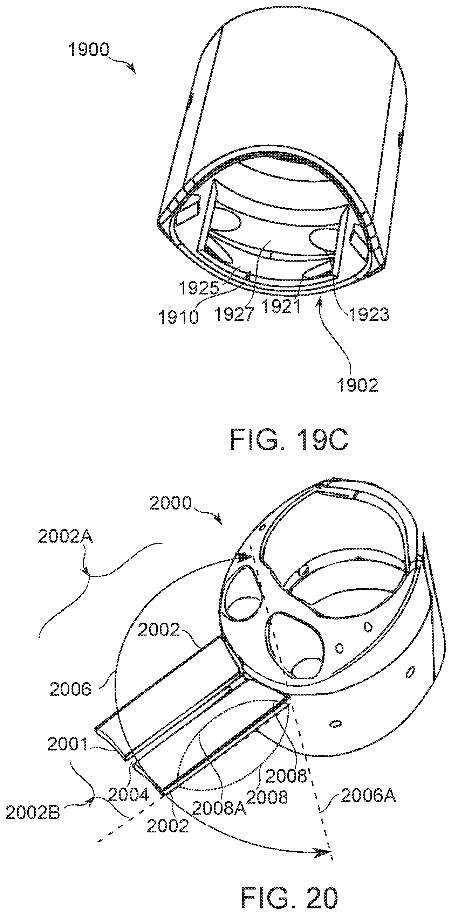

Reference is now made to *Figures 18A-18C**,* which schematically illustrate a tip adaptor **1800** comprising a shell **1801** and an insert **1805,** according to some exemplary embodiments of the invention. In some embodiments, the shell is relatively soft, and the insert is relatively hard. In some embodiments, the insert is relatively soft, and the shell is relatively hard. The two alternative configurations potentially provide alternative sets of benefits, as described hereinbelow. Reference is also made to *Figures 19A-19C**,* which schematically illustrate a tip adaptor **1900** comprising an evacuation antechamber **1910,** according to some exemplary embodiments of the invention.

In some embodiments of the invention, a tip adaptor **1800, 1900** comprises a colonoscope mounting lumen **1903,** sized and shaped to receive the distal end of a colonoscope. Optionally, the tip adaptor **1800, 1900** also comprises:
- one or more evacuation channel mounting sockets **1902** for tubes of the evacuation channel,
- one or more irrigation channel mounting sockets **1912** for tubes providing irrigation fluid, and/or
- one or more pressure sensor sockets **1918, 1914** for receiving pressure sensing means.

In some embodiments of the invention, an evacuation socket **1902** comprises structures related to the assembly, sensing, safety, and/or blockage resistance of the evacuation channels of a colon cleaning device tip adaptor.

In some embodiments, the distal end of the tube of an evacuation channel **22** inserts into the proximal side of an evacuation channel mounting socket **1902.** Optionally, it is secured by a friction fit and/or adhesive. Optionally, the depth of tube insertion is limited by a tube stop **1904,** which comprises a partial restriction within the lumen of evacuation socket **1902.** The partial restriction is optionally a circumferential narrowing of socket **1902.** Optionally, the stop comprises a non-circumferential raised portion of the lumen wall.

Distal to the tube stop, in some embodiments, an evacuation channel tip pressure sensing aperture **1909** is provided. Optionally, the pressure sensing aperture comprises a lumen extending between evacuation channel mounting socket **1902,** and pressure sensor socket **1918.** In some embodiments, a hole **1907** is provided opposite the sensing aperture hole **1909** in the wall of evacuation socket **1902.** The hole **1907** is optionally created during the process of manufacture to provide access for the creation of hole **1909.**

Additionally or alternatively, it serves as a secondary suction relief aperture.

In some embodiments, socket **1902** terminates at a distal evacuation socket aperture **1923,** leading into evacuation antechamber **1910.** As such, socket aperture **1923** also comprises the intake aperture **95** of the evacuation channel.

Optionally, a proximal wall **1927** of evacuation antechamber **1910** comprises one or more such distal evacuation socket apertures **1923.** In some embodiments, a distal wall **1925** of evacuation antechamber **1910** comprises one or more evacuation antechamber access apertures **1921.** Optionally, evacuation antechamber **1910** comprises another aperture **1910A,** across which lumen **1903** is in fluid communication with antechamber **1910.**

In some embodiments, the structures forming antechamber **1910** and its apertures comprise functions described also, for example, in relation to *Figures 3A-5B**,* and/or *7A-7C.* For example, distal wall **1925** comprises an intake guard **41,** insofar as it enforces a separation of the intestinal wall **1** from socket aperture **1923.** Evacuation antechamber access aperture **1921** comprises an embodiment of an aperture or hole **60** which allows passage of sufficiently small material therethrough.

Potential advantages of the structure of antechamber **1910** and its apertures can be considered in terms of the pressures experienced by different sizes of granulated waste material as it passes into the antechamber **1910,** and thence into one of the evacuation intake apertures **1923.** With the evacuation channel side of the cleaning probe oriented to down (as in, for example, *Figures 5A-5B*), fluid level is readily reduced to a low depth, as the access apertures **1921** are positioned low on the tip head, and a sufficient pressure gradient is generated by suction from the evacuation channel **22** across access apertures **1921** to move fluid volume.

Optionally, access apertures **1921** are sized to be no larger than intake apertures **1923.** Optionally, access apertures **1921** are positioned axially distal to intake apertures **1923.** A potential advantage of this is that it helps ensure that an oblate waste particle entering access aperture **1921** is oriented to enter the evacuation channel, rather than block across it. In this sense, access aperture **1921** potentially acts as a waste particle strainer. In cases where waste particles are too large, and/or improperly oriented to pass into an access aperture **1921,** the waste particle is nevertheless less likely to become suction-impacted within the access aperture **1921,** as the pressure gradient is relieved by one of the other antechamber apertures **1921, 1910A, 1907.** Aperture **1910A,** by being relatively elevated, and at a relatively protected position within lumen **1903,** is less likely to encounter large particles for which pre-straining is an advantage. Optionally, however, aperture **1910A** is divided into two or more smaller apertures, potentially also providing a straining function for the intake aperture **1923.**

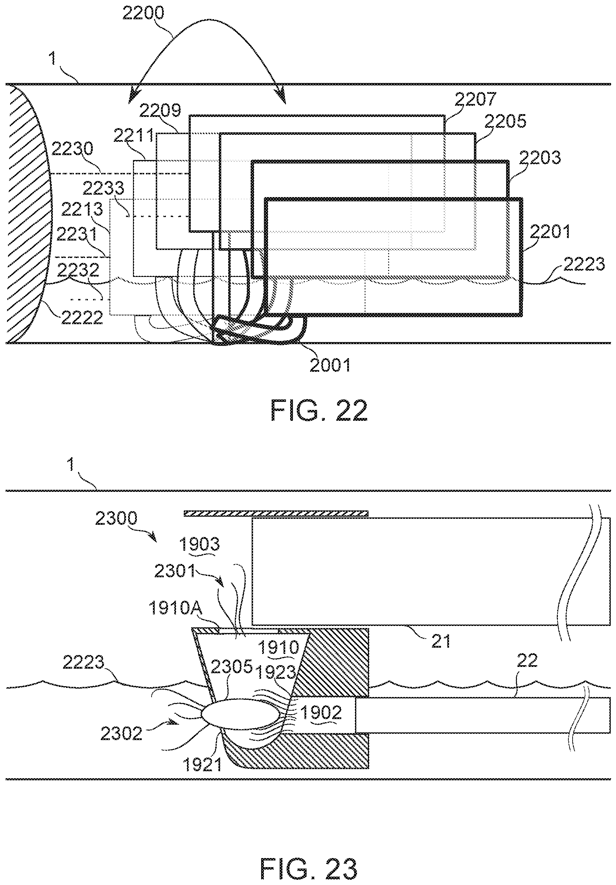

Reference is now made to *Figure 23**,* which schematically illustrates a particle **2305** entering a distal tip adaptor **2300,** according to some exemplary embodiments of the invention.

In some embodiments, a tip **2300** (simplified to emphasize aspects related to evacuation antechamber **1910**) is attached to a colonoscope probe **21** within lumen **1903,** and an evacuation channel **22** at evacuation socket **1902.** Suction is actively applied to the evacuation channel **22** such that material entering antechamber **1910** is evacuated. In some embodiments, this creates additional suction gradients **2301, 2302** from apertures **1910A** and **1921,** respectively. When waste (fluid with suspended solids) level **2223** is positioned about at or above aperture **1921,** waste is pulled into the antechamber. If resistance at this aperture rises, however, the corresponding rise in the steepness of the pressure gradient is reduced by suction relief action of aperture **1910A.** Resistance can rise, for example, upon encountering a portion of intestinal wall **1,** for example as shown in relation to *Figures 2D-2E**.* Potentially, shunting of the pressure gradient acts to reduce an incidence of suction-related injury. Particle **2305** represents a waste particle which, though large in one dimension, has been aligned to by passage through aperture **1921** so that it can enter channel **22** rather than being jammed to across intake aperture **1923.**

In some embodiments, the provision of pluralities of apertures provides a further functional advantage. If a single access aperture **1921** becomes blocked, for example, it is nevertheless possible for a plurality of evacuation intake apertures to continue receiving fluid from the common antechamber **1910** fed by another access aperture **1921.**

A further functional aspect related to evacuation antechamber **1910** is the rounded shaping of the distal wall **1925** such that while it provides separation of regions experiencing a steep pressure from the intestinal wall **1,** it also presents a blunted front aspect **1802.** Potentially, this reduces mechanical damage from poking, cutting, catching, and/or slicing. Insofar as the rounded tip shape is also tapering, the chances of catching are potentially reduced, as the shape of the tip encourages sliding away from and over wall obstructions, rather than bluntly pushing at them. Potentially, rounded shaping of the whole of the front surface ("spherical" shaping, though it need not be actually a section of a spherical surface) also reduces the likelihood of catching on walls by suction grip, by presenting inlet aperture shapes to which the intestinal wall 1 is not well suited to simultaneously conform (and block).

Another aspect of distal tip **1800, 1900** relates to the structures associated with colonoscope mounting lumen **1903.** In some embodiments, a colonoscope stop **1906** is provided, which protrudes into the lumen **1903** such that the placement of the cleaning tip **1800, 1900** on the colonoscope distal end is precisely limited to the point at which the colonoscope contacts stopping surface **1906A.** It is a potential advantage, for example, if the colonoscope end is prevented from blocking antechamber aperture **1910A.**

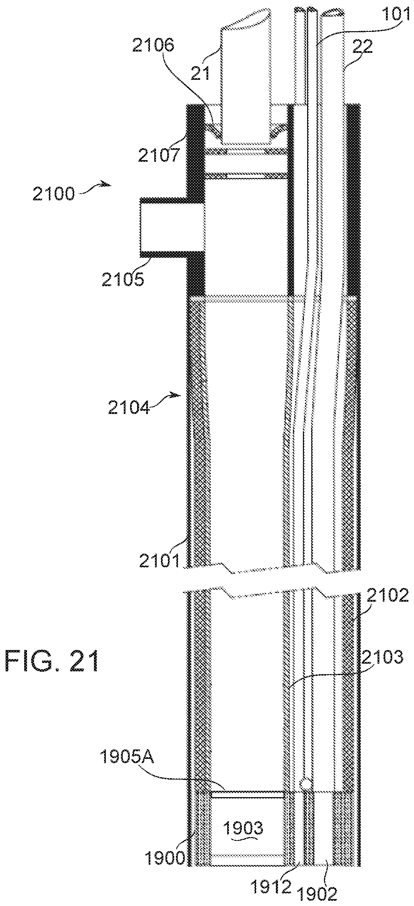

Reference is now made to *Figure 21**,* which schematically illustrates a sleeve assembly **2104** in a sleeve placement jig **2100** together with components of an irrigation system and a colonoscope **21,** according to some exemplary embodiments of the invention.

In some embodiments of the invention, tip **1800, 1900** is attached to one end of a thin-walled, flexible sleeve of a polymer (such as polyurethane, silicone rubber, or another flexible polymer) which is sized to fittingly accommodate and/or fair the colonoscope and/or other tube elements which comprise the fully assembled cleaning apparatus. Potential advantages of the sleeve assembly **2104** include: allowing reversible attachment of the cleaning apparatus to a colonoscope (for example, so that the cleaning apparatus can be exchanged and/or disposed of after use); ease of integration of a cleaning system with a colonoscope probe, and/or reproducible control of the integrated cleaning system/colonoscope probe system configuration shape and relative positioning.

In some embodiments, the sleeve assembly comprises an inner sleeve **2103,** adapted for receiving a colonoscope probe **21.** Inner sleeve **2103** is circumferentially surrounded by an outer sleeve **2102,** including one or more add-on tubes **21, 101.**

In some embodiments, a diameter of a lumen of inner sleeve **2103,** in a non-expanded state, ranges between, for example, 8-12 mm, 3-10 mm, 5-18 mm, or another range having the same, intermediate, larger and/or smaller bounds. Optionally, the diameter is selected according to a diameter of a colonoscope; for example, 5%, 10%, 20% or an intermediate, larger or smaller percentage smaller (before stretching) than a diameter of a colonoscope. As particular examples: for a colonoscope having a diameter of 12 mm, the inner diameter is smaller than the colonoscope's diameter in a non-expanded state by 0.1-5 mm, 1-3 mm, 0.1-0.9 mm or another range of sizes having the same, intermediate, larger and/or smaller bounds.

In some embodiments, sleeve assembly **2104** is inflatable for receiving a colonoscope probe by attachment to an inflation base **2107** of inflation jig **2100,** the base **2107** comprising an inflation inlet **2105** configured to be attached to a gas pressure source for inflation. Optionally, an inflation jig **2100** comprises a restriction tube or partial tube **2101,** though which sleeve assembly **2104** is extended for receiving a colonoscope probe **21.** Colonoscope probe **21** is optionally inserted through sealing member **2106,** and fed through the inner sleeve **2103** until reaching lumen **1903** of the distal tip **1900.**

Optionally, the flexible sleeve is secured by a circumference around attachment channel **1905A.** Optionally, the sleeve comprises an outer tube and at least one inner tube; optionally, the inner tube is attached to attachment channel **1905A.** Optionally, adhesive introduction aperture **1905** is provided, leading from the outside of the tip leading to lumen **1903** and attachment channel **1905A.** Optionally, the fairing tube is secured to channel **1905A** by placement of one end within lumen **1903** and pressing against channel **1905A,** followed by adhesive injection all around the lumen. Returning to *Figures 18A-19C**,* in some embodiments of the invention, irrigation channel **101** terminates at tip **1800, 1900** by insertion into an irrigation tube receiving socket **1912.** Optionally, socket **1912** is elongated to allow two or more separate irrigation tubes to insert alongside each other. Optionally, insert depth into the socket is set by one or more irrigation tube stops **1913.** In some embodiments, one or more irrigation outlets **1920** are provided for each socket **1912.** Optionally, the irrigation outlets **1920** comprise cylindrical holes oriented along the distal-proximal axis of the tip body. Optionally, the irrigation outlets **1920** are otherwise shaped and/or oriented for jetting-for example, comprising a tapered lumen, and/or aimed off the distal-proximal axis for distributing and/or aiming the irrigation fluid spray.

In some embodiments of the invention, sensor sockets **1918, 1914** are configured to receive sensing probe and/or sensing probe portions, to allow one or more aspects of the tip and/or tip environment to be sensed. For example, evacuation lumen pressure sensor socket **1918** is optionally provided with a sensor which is linked through sensing aperture **1909** to one or more evacuation sockets **1902** for sensing of pressure and/or pressure changes therein. In some embodiments, an electronic pressure sensor is provided. In some embodiments, a mechanical pressure sensor is provided. For example, a pressure sensor optionally comprises a fluid-filled tube to which a test pressure is proximally applied. As pressure at the distal tip rises and/or falls, the volume moved by the test pressure changes, which is optionally transduced into a measurement and/or indication of distal tip pressure.

In some embodiments, external pressure sensor socket **1914** is configured to receive a pressure sensor which senses an external pressure through external pressure sensor aperture **1916.** Optionally, a stop **1915, 1919** helps to assure proper placement of the sensor, for example, a mechanical pressure sensor such as that described in relation to pressure sensing socket **1918.**

In some embodiments of the invention, a tip assembly **1800** comprises a relatively hard shell **1801,** with a softer mounting portion **1805.** Optionally, mounting portion **1805** comprises an insert into a receiving aperture **1801A** of hard shell **1801.** Potentially, a soft insert/hard shell configuration provides an advantage by allowing the material to be selected according to the functions performed by each portion **1801, 1805.** For example, a hard shell potentially provides greater dimensional stability to the tip adaptor overall. Potentially, this encourages the tip to slide over rather than deformingly embed within intestinal tissue that it encounters, an advantage for navigation and/or safety, for example. Potentially, a soft insert provides an advantage for resisting the tendency of tubes inserted thereto from being pried out of position upon application of bending forces to the cleaning assembly (if made to a softness similar to the tubes **110,** the insert bends to a greater degree along with the tubes themselves as they are flexed).

Another potential advantage of the two-hardness construction is to enhance elastic friction fitting of the various small tubes which connect to the tip; for example, by making sockets **1912, 1914, 1902,** and/or **1918** slightly smaller than the tubes which are pressingly fitted into them. Potentially, the hard shell provides an advantage for allowing a relatively lower friction material (of the body itself, and/or of a coating) to be used for regions of the tip liable to contact the intestinal wall 1. Optionally, both components are made of polyurethane (of appropriately differing hardness). Optionally, the outer component is coated, for example with a friction reducing material such as Parylene C, Teflon, or another low-friction material.

In some embodiments, the hardness of the internal tip portion **1805** is, for example, within the range of 25-40 Shore A, 30-50 Shore A, 35-55 Shore A, or within another range of softness having the same, larger, smaller, and/or intermediate bounds.

In some embodiments, the hardness of the external tip portion **1801** is, for example, within the range of 40-60 Shore A, 60-80 Shore A, 70-90 Shore A, or within another range of softness having the same, larger, smaller, and/or intermediate bounds.

In some embodiments of the invention, a tip assembly **1800** comprises a relatively soft shell **1801,** with a harder mounting portion **1805.** Optionally, mounting portion **1805** comprises an insert into a receiving aperture **1801A** of soft shell **1801.** Potentially, a soft shell/hard insert configuration provides an advantage by allowing the material to be selected according to the functions performed by each portion **1801, 1805,** with a different emphasis than described for the soft insert/hard shell embodiments hereinabove.

For example, a hard insert potentially provides dimensional stability to the tip adaptor interfaces with tubing connectors such as sockets **1902, 1912, 1914, 1918,** and/or to the dimensions of apertures such as apertures **1920** which optionally direct and/or shape fluid jets. Potentially, a hard insert is manufacturable to closer tolerances than a soft insert, for example to achieve tighter fitting, and/or closer control of jet aperture characteristics. During storage, a hard insert potentially resists creep, sagging, or other deformation which could potentially affect performance. During use, dimensional stability potentially prevents forces exerted on the tip from distorting, dislodging and/or loosening socket-tube connections. Potentially, dimensional stability of fluid jet apertures prevents distortion which affects the aiming and/or jet forming characteristics of the aperture.

In some embodiments, a soft shell protects tissue from encounters with hard portions of the tip. In general, providing a soft "skin" for the shell **1801** potentially helps to prevent damaging effects of poking and/or scraping. For example, a soft construction for shell **1801** softens the edges around apertures such as colonoscope mounting lumen **1903,** irrigation outlet **1920,** and/or evacuation socket aperture **1923.** A soft shell **1801** also potentially distributes contact forces at other parts of the tip assembly **1800,** for example at outer corners and/or curves.

In some embodiments, one or more compressible chambers and/or hollows are provided which potentially augment the protective effects of a soft shell **1801.** For example, antechamber **1910** is optionally manufactured of material of sufficient softness and/or wall thinness such that the hollow it defines is easily collapsible upon encountering contact forces.

In some embodiments, the antechamber **1910** is flexible enough to allow elastic collapse with light pressure (for example, a pressure of between 1-10 Newtons, or another greater or smaller pressure). The collapse pressure is optionally chosen to be light enough that it is unlikely to damage the colon and/or at a level which is ordinarily encountered during advance within the colon. Optionally, collapsing pressure is deliberately brought to bear on the antechamber **1910** during use. Potentially, the collapsing action allows mechanically clearance of blockages within the chamber, and/or at one of the entrances to/exits from the chamber.

Additionally or alternatively, shell **1801** is manufactured with other hollows within its structure. For example, the wall thickness of a shell region **1803** comprises hollow regions defined by relatively thin membranes of soft material (in a honeycomb formation, for example). The chambers are optionally sealed or left unsealed on at least one side.

In some embodiments, the regions of soft and hard material are provided in different regions than shown in the figures. For example, the soft material of shell **1801** optionally comprises nearly all of the material volume of a tip assembly **1800,** with the dimensionally stable hard material being provided as one or more inserts (for example, a plurality of tubular inserts) to define structures such as sockets and/or apertures. Some embodiments of the invention comprise another alternative construction; for example, a plurality of alternating and/or intermingled soft and hard layers and/or partial or segmented shells. In some embodiments, at least a portion of the relative advantages of each type of two-hardness construction (soft inside, hard outside; hard inside, soft outside) is potentially obtained by such construction variations. For example, hard sockets and/or jet apertures (potentially providing dimensional stability for connection/jet formation) are optionally embedded in a softer matrix (potentially allowing parts to move with respect to one another without being dislodged thereby); the softer matrix is optionally embedded in a harder outer part (optionally helping to preserve overall strength and/or shape of the adaptor), and the harder outer part itself is optionally provided with soft regions which act as force-absorbing and/or edge shielding bumpers.

Optionally, one or both of the soft and hard components are made of polyurethane (of appropriately differing hardness). Optionally, the outer component is coated, for example with a friction reducing material such as Parylene C, Teflon, or another low-friction material.

In some embodiments, the hardness of the shell portion **1801** is, for example, within the range of 25-40 Shore A, 30-50 Shore A, 35-55 Shore A, or within another range of softness having the same, larger, smaller, and/or intermediate bounds. In some embodiments, the hardness of the insert tip portion **1805** is, for example, within the range of 40-60 Shore A, 60-80 Shore A, 70-90 Shore A, or within another range of softness having the same, larger, smaller, and/or intermediate bounds.

Reference is now made to *Figure 20**,* which schematically illustrates a tip adaptor **2000** comprising a flexible wall guard **2001,** according to some exemplary embodiments of the invention.

In some embodiments of the invention, a tip adaptor **2000** comprises a flexible wall guard **2001,** which acts as a spacing, positioning and/or aiming aid during colon cleaning operations.

In some embodiments, the wall guard **2001** comprises one or more flexible members **2002** attached at one end to the tip body, and having a length **2002A** of about, for example, 10-15 mm, 12-18 mm, 15-20 mm, 18-24 mm, or another range of lengths having the same, larger, smaller, and/or intermediate bounds. In some embodiments, the total width **2002B** of the wall guard **2001** is within the range of about, for example, 5-7 mm, 6-9 mm, 8-12 mm, 9-14 mm, or another range having the same, larger, smaller, and/or intermediate bounds. Optionally, a plurality of flexible members are separated by a gap **2004;** for example, a gap of 1-2 mm or another gap width.

In some embodiments, the members of wall guard **2001** are sufficiently flexible to fully bend through a range **2006** of about 180° or more. In some embodiments, the maximum bending range is, for example, about 150°, 160°, 170°, 180°, 190°, or another larger, smaller, or intermediate maximum range. In some embodiments, the wall additionally or alternatively is configured to flex along its length, for example through a flexing range **2008.** In some embodiments, the maximal flexing range is optionally from about doubled over (180°), or a smaller range such as about 90°, about 45°, or another maximum range which is larger, smaller, or intermediate. A paired-rectangle "rabbit ear" configuration is shown in *Figure 20**;* however, it is to be understood that other shapes are also embodiments of the invention-for example, triangular, oval, semi-circular, or another shape. In some embodiments of the invention, the thickness of the wall guard members is varied according to distance from the tip, in order to encourage more or less bending along the length, according to the specific bending characteristics desired. The hardness of the wall guard members **2002** (which comprise, for example, polyurethane or another rubber polymer) are, for example, about 20-30 Shore A, 30-45 Shore A, 40-55 Shore A, or within another range of hardness having the same, larger, smaller, and/or intermediate bounds. It is to be understood that the wall guard **2001** is optionally provided with any of the cleaning system tip embodiments described herein. Optionally, the wall guard **2001** is integrally formed with a soft outer shell such as shell **1801,** optionally surrounding an insert such as mounting portion **1805.**

Reference is now made to *Figure* 22, which illustrates positions **2201, 2203, 2205, 2207, 2209, 2211, 2213** of a cleaning system distal region (comprising for example, an adaptor tip **2100**), in relation to an intestinal wall 1 and a flexible wall guard **2001.**

Wall guard **2001** provides a potential advantage by acting as a soft (atraumatic) stand-off from the intestinal wall through which a cleaning system tip **2000** navigates. Potentially, this reduces instances of suction attachment to the intestinal wall.

Nevertheless, the wall guard **2001** is optionally sufficiently soft that it tends to elastically collapse upon exercise of sufficient force, for example, upon encountering a restriction in the colon (for example, a constriction at a colon segment boundary), such that navigation through the restriction is relatively unimpeded. Positions **2200** and **2213** show examples of the wall collapsed in a distal- and proximal-pointing orientation, respectively. In some embodiments, the members **2002** are provided as balloons inflatable through fluid communication with a pressure source (such as an irrigation tube **101,** or another tube which is specially provided for wall inflation). This optionally allows control of wall stiffness according to the current navigational requirements.

In some embodiments of the invention, the wall guard **2001** serves as a device that helps to position the tip radially within the colon. For example: as the tip is advanced from position **2207,** wall guard members **2002** tend to flex backward and toward the tip body to a position approximating that of line **2006A** and/or position **2213,** such that the tip can more closely approach the intestinal wall **1.** However, with a slight backward pull, the members **2002** tend to straighten, for example, toward position **2008A** and/or **2207.** This potentially pushes the tip away from the wall. An optional alternative maneuver is to bend the wall forward by withdrawing the tip proximally; from this position, advancing rises up on the walls (sequence of positions **2201, 2203, 2205, 2207,** for example). By this means, different radial positions for the tip are selectable, which potentially allows selection of the position at which jets are aimed when irrigation fluid is supplied-for example the difference in position between jet aiming directions **2230** and **2231.**

Additionally or alternatively, the tip is repositioned by the wall guard to a radial position better suited to further advancement-for example, nearer to an aperture region in an intestinal restriction. Optionally, alternate short backward and forward movements of the tip region (for example, between positions **2201** and **2213**) result in the tip "scanning" within the lumen and toward and away from the intestinal wall **1** (paralleling, for example, a path like **2200**). This potentially distributes the energy of irrigation jets over a cleaning target **2222,** and/or allows selection of a position suited for draining fluid from the intestine relative to a waste level **2223** (allowing, for example, positioning an evacuation intake zone between levels **2232** and **2233**). An evacuation position is optionally selected, for example, such that the evacuation access apertures **1921** are immersed, but the evacuation antechamber aperture **1910A** remains clear of fluid.

Optionally, rotation of the device puts more force on one of the wall members **2002** than on the other, as another method of guard-wall mediated steering. Optionally, the device is deliberately rotated by about 90° to move the guard wall out of the way and bring a portion of an evacuation access aperture nearer to a lower intestinal wall **1** portion, such that small amounts of remaining fluid can be evacuated.

As used herein, the term "about" refers to within ±10%.

The terms "comprises", "comprising", "includes", "including", "having" and their conjugates mean: "including but not limited to".

The term "consisting of" means: "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

The words "example" and "exemplary" are used herein to mean "serving as an example, instance or illustration". Any embodiment described as an "example or "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features except insofar as such features conflict.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6, etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

In addition, citation or identification of any reference in this application shall not be construed as an admission that such reference is available as prior art to the present invention. To the extent that section headings are used, they should not be construed as necessarily limiting.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

## Claims

1. A tip adaptor (1800, 1900) with dimensionally stable sockets, for use with a colonoscope probe in a colon cleaning system, the tip adaptor comprising a shell (1801) and an insert (1805):
the shell having a hollow region sized to fittingly accommodate the insert, and at least one mounting lumen (1903) sized and shaped to receive a distal end of a colonoscope probe therein;
said tip adaptor **characterized in that**:
the insert is relatively rigid and comprises a plurality of sockets (1902, 1912), each socket being configured to fittingly accommodate the distal end of one or more respective fluid transport tubes; and
the shell comprises an elastic component of a softer material than the insert, the shell being deformable around the insert in response to external force, while the insert remains in place and substantially undeformed;
such that said plurality of sockets are made dimensionally stable by said rigid insert while being protected by a surrounding portion of said shell.

2. The tip adaptor of claim 1, wherein the soft shell defines at least one elastically collapsible hollow.

3. The tip adaptor of claim 2, wherein the collapsible hollow comprises a guard wall which extends across and is spaced from an aperture of at least one of the sockets along a longitudinal axis of the socket.

4. The tip adaptor of claim 3, wherein the guard wall comprises an aperture of the collapsible hollow.

5. The tip adaptor of claim 3, wherein the aperture of the socket is a suction intake aperture.

6. The tip adaptor of claim 5, wherein the aperture of the guard wall has substantially the same size and shape as the suction intake aperture.

7. The tip adaptor of claim 3, wherein the guard wall is spaced from the aperture along a longitudinal axis of the socket by at least 5 mm.

8. The tip adaptor of claim 2, wherein the collapsible hollow is sufficiently flexible to elastically collapse upon being pressed from within against a portion of colon wall, the collapse occurring at a force below one that damages the portion of colon wall.

9. The tip adaptor of claim 1, wherein the shell comprises an aperture surrounding an aperture of at least one of the sockets.

10. The tip adaptor of claim 9, wherein the aperture of the socket is an irrigation aperture.

11. The tip adaptor of claim 10, wherein the irrigation aperture is shaped to form fluid into a jet upon the supply of fluid therethrough, and the aperture of the shell is sufficiently large to avoid interference with the jet.

12. The tip adaptor of claim 1, wherein the tip adaptor comprises a socket sized to receive a distal portion of the colonoscope probe.

13. A system for use with a colonoscope probe, comprising:
an evacuation channel sized for insertion to a distal segment of a colon; and
the tip adaptor of claim 1, wherein the tip adaptor is attached to the distal end of the evacuation channel.

14. The tip adaptor of claim 1, comprising a colon spacer integrally formed with the shell, attached to a circumference of the tip adaptor and extending radially therefrom;
the colon spacer being sufficiently flexible that it collapses upon receiving pressure due to forward motion of the tip adaptor into a radially restricted region of colon.

15. The tip adaptor of claim 1, wherein the hardness of the insert is between 40-90 Shore, and the hardness of the shell is between 25-55 Shore.

## Patentansprüche

1. Spitzenadapter (1800, 1900) mit formstabilen Buchsen zur Verwendung mit einer Koloskopsonde in einem Darmreinigungssystem, wobei der Spitzenadapter eine Hülse (1801) und einen Einsatz (1805) umfasst:
wobei die Hülse eine hohle Region, die dazu bemessen ist, den Einsatz passend unterzubringen, und mindestens ein Montagelumen (1903), das dazu bemessen und geformt ist, ein distales Ende einer Koloskopsonde darin aufzunehmen, aufweist;
wobei der besagte Spitzenadapter **dadurch gekennzeichnet ist, dass**:
der Einsatz relativ starr ist und eine Vielzahl von Buchsen (1902, 1912) umfasst, wobei jede Buchse dazu konfiguriert ist, das distale Ende eines oder mehrerer jeweiliger Flüssigkeitstransportschläuche passend unterzubringen;
die Hülse eine elastische Komponente aus einem weicheren Material als der Einsatz umfasst, wobei die Hülse als Reaktion auf eine externe Kraft um den Einsatz verformbar ist, während der Einsatz in Position und im Wesentlichen unverformt bleibt;
so dass die besagte Vielzahl von Buchsen durch den besagten starren Einsatz formstabil gemacht wird, während sie durch einen umschließenden Teil der besagten Hülse geschützt wird.

2. Spitzenadapter nach Anspruch 1, wobei die weiche Hülse mindestens einen elastisch kollabierbaren Hohlraum definiert.

3. Spitzenadapter nach Anspruch 2, wobei der kollabierbare Hohlraum eine Schutzwand umfasst, die sich über eine Öffnung mindestens einer der Buchsen entlang einer Längsachse der Buchse erstreckt und davon beabstandet ist.

4. Spitzenadapter nach Anspruch 3, wobei die Schutzwand eine Öffnung des kollabierbaren Hohlraums umfasst.

5. Spitzenadapter nach Anspruch 3, wobei die Öffnung der Buchse eine Ansaugöffnung ist.

6. Spitzenadapter nach Anspruch 5, wobei die Öffnung der Schutzwand im Wesentlichen dieselbe Größe und Form wie die Ansaugöffnung aufweist.

7. Spitzenadapter nach Anspruch 3, wobei die Schutzwand um mindestens 5 mm von der Öffnung entlang einer Längsachse der Buchse beabstandet ist.

8. Spitzenadapter nach Anspruch 2, wobei der kollabierbare Hohlraum ausreichend flexibel ist, um elastisch zu kollabieren, wenn aus dem Inneren gegen einen Teil der Darmwand gedrückt wird, wobei das Kollabieren bei einer Kraft erfolgt, die niedriger als eine Kraft ist, die den Teil der Darmwand beschädigt.

9. Spitzenadapter nach Anspruch 1, wobei die Hülse eine Öffnung umfasst, die eine Öffnung mindestens einer der Buchsen umschließt.

10. Spitzenadapter nach Anspruch 9, wobei die Öffnung der Buchse eine Spülöffnung ist.

11. Spitzenadapter nach Anspruch 10, wobei die Spülöffnung dazu geformt ist, bei der Zufuhr von Flüssigkeit dort hindurch einen Strahl aus Flüssigkeit zu bilden, und die Öffnung der Hülse ausreichend groß ist, um eine Störung des Strahls zu vermeiden.

12. Spitzenadapter nach Anspruch 1, wobei der Spitzenadapter eine Buchse umfasst, die dazu bemessen ist, einen distalen Teil der Koloskopsonde aufzunehmen.

13. System zur Verwendung mit einer Koloskopsonde, umfassend:
einen Entleerungskanal, der zur Einführung in ein distales Segment eines Darms bemessen ist; und
den Spitzenadapter nach Anspruch 1, wobei der Spitzenadapter an dem distalen Ende des Entleerungskanals angebracht ist.

14. Spitzenadapter nach Anspruch 1, umfassend einen Darmabstandshalter, der mit der Hülse integral ausgebildet ist, an einem Umfang des Spitzenadapters angebracht ist und sich davon radial erstreckt;
wobei der Darmabstandshalter ausreichend flexibel ist, so dass er bei Erhalten von Druck aufgrund einer Vorwärtsbewegung des Spitzenadapters in einer radial begrenzten Region des Darms kollabiert.

15. Spitzenadapter nach Anspruch 1, wobei die Härte des Einsatzes zwischen 40 und 90 Shore liegt und die Härte der Hülse zwischen 25 und 55 Shore liegt.

## Revendications

1. Adaptateur d'embout (1800, 1900) comportant des douilles dimensionnellement stables, destiné à une utilisation avec une sonde de colonoscope dans un système de nettoyage du côlon, l'adaptateur d'embout comprenant une coque (1801) et un insert (1805) :
la coque comportant une région creuse dimensionnée pour recevoir par emboîtement l'insert, et au moins une lumière de montage (1903) dimensionnée et formée pour y recevoir une extrémité distale d'une sonde de colonoscope ;
ledit adaptateur d'embout étant **caractérisé en ce que** :
l'insert est relativement rigide et comprend une pluralité de douilles (1902, 1912), chaque douille étant configurée pour recevoir par emboîtement l'extrémité distale d'un ou plusieurs tubes de transport de fluide respectifs ; et
la coque comprend un composant élastique constitué d'un matériau plus souple que l'insert, la coque pouvant être déformée autour de l'insert en réponse à une force externe, tandis que l'insert reste en place et n'est pratiquement pas déformé ;
de telle sorte que ladite pluralité de douilles soit rendue dimensionnellement stable par ledit insert rigide tout en étant protégée par une partie de ladite coque dont elle est entourée.

2. Adaptateur d'embout selon la revendication 1, dans lequel la coque souple délimite au moins un creux élastiquement affaissable.

3. Adaptateur d'embout selon la revendication 2, dans lequel le creux affaissable comprend une paroi de protection qui s'étend à travers, et est espacée de, une ouverture d'au moins une des douilles le long d'un axe longitudinal de la douille.

4. Adaptateur d'embout selon la revendication 3, dans lequel la paroi de protection comprend une ouverture du creux affaissable.

5. Adaptateur d'embout selon la revendication 3, dans lequel l'ouverture de la douille est une ouverture d'entrée d'aspiration.

6. Adaptateur d'embout selon la revendication 5, dans lequel l'ouverture de la paroi de protection a sensiblement la même taille et la même forme que l'ouverture d'entrée d'aspiration.

7. Adaptateur d'embout selon la revendication 3, dans lequel la paroi de protection est espacée d'au moins 5 mm de l'ouverture le long d'un axe longitudinal de la douille.

8. Adaptateur d'embout selon la revendication 2, dans lequel le creux affaissable est suffisamment flexible pour s'affaisser élastiquement lorsqu'il est pressé de l'intérieur contre une partie de la paroi du côlon, l'affaissement se produisant à une force inférieure à une force qui endommagerait la partie de la paroi du côlon.

9. Adaptateur d'embout selon la revendication 1, dans lequel la coque comprend une ouverture entourant une ouverture d'au moins une des douilles.

10. Adaptateur d'embout selon la revendication 9, dans lequel l'ouverture de la douille est une ouverture d'irrigation.

11. Adaptateur d'embout selon la revendication 10, dans lequel l'ouverture d'irrigation est formée pour former un fluide en un jet lorsque le fluide est amené à passer à travers, et l'ouverture de la coque est suffisamment grande pour éviter une interférence avec le jet.

12. Adaptateur d'embout selon la revendication 1, l'adaptateur d'embout comprenant une douille dimensionnée pour recevoir une partie distale de la sonde de colonoscope.

13. Système destiné à une utilisation avec une sonde de colonoscope, comprenant :
un canal d'évacuation dimensionné pour une insertion dans un segment distal d'un côlon ; et
l'adaptateur d'embout selon la revendication 1, l'adaptateur d'embout étant fixé à l'extrémité distale du canal d'évacuation.

14. Adaptateur d'embout selon la revendication 1, comprenant un espaceur de côlon formé en une seule pièce avec la coque, fixé à une circonférence de l'adaptateur d'embout et s'étendant radialement à partir de celle-ci ;
l'espaceur de côlon étant suffisamment flexible pour s'affaisser lorsqu'il reçoit une pression due à un avancement de l'adaptateur d'embout le faisant pénétrer dans une région radialement limitée du côlon.

15. Adaptateur d'embout selon la revendication 1, dans lequel la dureté de l'insert est comprise entre 40 et 90 Shore et la dureté de la coque est comprise entre 25 et 55 Shore.
